(19) Europäisches Patentamt
European Patent Office
Office européen des brevets

(11) **EP 4 500 173 B1**

(12) **EUROPÄISCHE PATENTSCHRIFT**

(45) Veröffentlichungstag und Bekanntmachung des Hinweises auf die Patenterteilung:
**06.05.2026 Patentblatt 2026/19**

(21) Anmeldenummer: **23712477.1**

(22) Anmeldetag: **15.03.2023**

(51) Internationale Patentklassifikation (IPC):
*G01N 33/18* *(2006.01)*

(52) Gemeinsame Patentklassifikation (CPC):
**G01N 33/1853; C02F 1/008;** C02F 1/42;
C02F 1/4691; C02F 2001/425; C02F 2209/05;
C02F 2209/055

(86) Internationale Anmeldenummer:
**PCT/EP2023/056579**

(87) Internationale Veröffentlichungsnummer:
**WO 2023/186539 (05.10.2023 Gazette 2023/40)**

(54) **VERFAHREN ZUR BESTIMMUNG DER HÄRTE VON WASSER UND VORRICHTUNG ZUR ERFASSUNG DER HÄRTE VON WASSER**

METHOD FOR DETERMINING THE HARDNESS OF WATER AND DEVICE FOR DETECTING THE HARDNESS OF WATER

PROCÉDÉ DE DÉTERMINATION DE LA DURETÉ DE L'EAU ET DISPOSITIF DE DÉTECTION DE LA DURETÉ DE L'EAU

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **30.03.2022 DE 102022107574**

(43) Veröffentlichungstag der Anmeldung:
**05.02.2025 Patentblatt 2025/06**

(73) Patentinhaber: **Grünbeck AG**
**89420 Höchstädt a.d. Donau (DE)**

(72) Erfinder:
• **MEINARDUS, Martin**
**89231 Neu-Ulm (DE)**
• **D'AMICO, Andrè**
**86462 Langweid (DE)**
• **WIEMANN, Alexander**
**74564 Crailsheim (DE)**

(74) Vertreter: **Charrier Rapp & Liebau**
**Patentanwälte PartG mbB**
**Fuggerstraße 20**
**86150 Augsburg (DE)**

(56) Entgegenhaltungen:
DE-A1- 102016 100 195     DE-A1- 4 114 380
DE-B3- 102007 059 058     DE-U1- 202011 102 629
US-A1- 2010 164 515       US-A1- 2014 231 348

**Beschreibung**

Die Erfindung betrifft ein Verfahren zur Bestimmung der Härte von Wasser, eine Vorrichtung zur Erfassung der Härte von Wasser sowie die Verwendung der Vorrichtung in einer Wasserbehandlungsanlage.

Die Summe der Konzentrationen sämtlicher gelöster Erdalkalimetalle (die als Carbonate bzw. Hydrogencarbonate, Sulfate, Chloride, Nitrite, Nitrate und Phosphate vorliegen können) wird dabei als Gesamthärte bezeichnet. Der nur an Kohlensäure gebundene Anteil wird als Carbonathärte (oder temporäre Härte) und die Differenz von Gesamthärte und Carbonathärte als Nichtcarbonathärte (oder permanente Härte) bezeichnet, wobei der überwiegende Teil der Gesamthärte im Trinkwasser in der Regel als Carbonathärte vorliegt. Als sogenannte Härtebildner tragen im Wesentlichen Calcium- und Magnesium-Ionen zur Gesamthärte von Wasser bei. Die Summe der Konzentration von Calcium- und Magnesium-Ionen entspricht daher in guter Näherung der Gesamtwasserhärte. Die übrigen Erdalkalimetalle, wie Strontium und Barium, sind regelmäßig nur als Spurenstoffe im Wasser vorhanden und tragen daher kaum zur Wasserhärte bei. Die Carbonathärte kann durch Beseitigung von Calcium- und Magnesiumcarbonat aus dem Wasser entfernt werden. Die gelösten Härtebildner Calcium und Magnesium können im Wasser schwer lösliche Verbindungen ausbilden, insbesondere als Carbonate mit dem im Wasser gelösten Kohlendioxid (gemäß dem pH-Wertabhängigen Kalk-Kohlensäure-Gleichgewicht).

Die Ausbildung von in Wasser schwer löslichen Verbindungen durch die Härtebildner führt insbesondere beim Erhitzen des Wassers zur Bildung von Kesselstein in Haushaltsgeräten, Heizungen und Heißwasserbereitern und vermindert die Wirksamkeit von Spül- und Waschmitteln in (Geschirr)-Spülmaschinen und Waschmaschinen. Weiterhin beeinflusst die Wasserhärte den Geschmack von mit Wasser zubereiteten Speisen und Getränken. Zur Vermeidung oder Minderung dieser nachteiligen Folgen von hartem Wasser, d.h. Wasser mit einer hohen Konzentration von Erdalkalimetall-Ionen, werden dem Wasser bspw. in Enthärtungsanlagen oder in Anlagen zur Vollentsalzung die Calcium- und Magnesiumionen teilweise oder vollständig entzogen, indem diese bspw. mittels Kationenaustauscher durch Natriumionen ersetzt oder - bei einer Vollentsalzung - zusammen mit allen anderen gelösten Ionen vollständig aus dem Wasser entfernt werden, bspw. durch eine Kombination von Kationen- und Anionenaustauscher oder durch eine Umkehrosmose. Für den Betrieb und die Steuerung von Enthärtungs- oder Entsalzungsanlagen ist die Kenntnis der Wasserhärte des zugeführten Rohwassers und/oder des enthärteten oder entsalzten Wassers oder der Härte von Verschnittwasser, das durch Mischung von hartem Rohwasser mit enthärtetem oder entsalztem Wasser erzeugt wird, von Bedeutung, um bspw. eine gewünschte Sollhärte des von einer Enthärtungsanlage bereitgestellten Wassers zu erzielen oder um einen Härtedurchbruch bei einer Erschöpfung der Ionenaustauscher zu verhindern.

Zur genauen Bestimmung der Wasserhärte können komplexometrische Titrationsmethoden, bspw. mit dem Dinatriumsalz der Ethylendiamintetraessigsäure (EDTA) als Titranten, eingesetzt werden, welche die Konzentrationen der im Wasser gelösten Erdalkali-Ionen und damit die Gesamthärte des Wassers erfassen können. Hierfür sind Messgeräte bekannt, welche den Farbumschlagspunkt der Titration fotometrisch erfassen. Diese Messgeräte zeichnen sich durch eine gute Messgenauigkeit aus, sind aber aufwendig und teuer in der Herstellung und erfordern eine regelmässige Wartung und Erzeugen wegen der Verwendung des Titranten hohe Kosten und werden daher kaum in automatisierten Messverfahren eingesetzt.

Weiterhin kann die Gesamthärte von Wasser durch ionenselektive Elektroden (ISE) bestimmt werden, welche die Ionenaktivität der Calcium- und Magnesiumionen erfassen. Ionenselektive Sensoren erfordern eine regelmäßige Kalibrierung mit einer Kalibrierflüssigkeit, wodurch die Anwendung in automatisierten und wartungsarmen Messverfahren erschwert wird.

Neben der Titrationsmethode und der Verwendung ionenselektiver Elektroden zur Bestimmung der Gesamthärte von Wasser, welche unmittelbar die Konzentration der Erdalkali-Ionen im Wasser erfassen, sind auch indirekte Messverfahren für die Bestimmung der Wasserhärte bekannt. So kann z.B. aus der elektrischen Leitfähigkeit des Wassers auf die Wasserhärte geschlossen werden. Da zur elektrischen Leitfähigkeit des Wassers jedoch neben den härtebildenden Calcium- und Magnesium-Ionen auch alle anderen im Wasser gelöste Ionen beitragen, ist diese Methode jedoch sehr unspezifisch, insbesondere wenn das Wasser einen hohen Anteil an Nichtcarbonathärte, bspw. bedingt durch einen hohen Gehalt an Chloriden, aufweist. Der Zusammenhang zwischen der elektrischen Leitfähigkeit und der Gesamthärte des Wassers, ist dabei sehr schwach und muss anhand von Kennlinien erfasst werden, indem der Zusammenhang zwischen der titrimetrisch bestimmten Härte von verschiedenen Wasserproben und deren elektrischer Leitfähigkeit bestimmt wird. Darüber hinaus ist die elektrische Leitfähigkeit von Wasser stark von der Temperatur abhängig, weshalb zusätzlich zur Leitfähigkeit die Temperatur erfasst und die Härtebestimmung über die Kennlinie eine Temperaturkorrektur erhalten muss. Daher eignen sich Leitfähigkeitsmessungen, welche den absoluten Leitwert einer Wasserprobe erfassen, nur bedingt zur Bestimmung der Gesamthärte der Wasserprobe.

Häufig werden allerdings Differenzmessungen der elektrischen Leitfähigkeit von Wasserproben oder eine Erfassung einer Änderung der elektrischen Leitfähigkeit der Wasserprobe nach einer Behandlung eingesetzt, um die Härte des Wassers zu bestimmen. Aus der EP 2 169 392 B1 ist ein Verfahren zur Bestimmung der Härte von Wasser bekannt, bei dem mindestens zwei Werte der elektrischen Leitfähigkeit einer Wasserprobe unter verschiedenen

**EP 4 500 173 B1**

Bedingungen gemessenen und die Härte des Wassers der Wasserprobe aus den Messwerten der elektrischen Leitfähigkeit bestimmt wird, wobei die Wasserprobe mittels einer ersten Verteilstation in mindestens zwei Fraktionen aufgeteilt und mindestens eine der Fraktionen einer Behandlung unterzogen wird, welche die elektrische Leitfähigkeit des Wassers beeinflussen kann. Nach der Behandlung der mindestens einen Fraktion, welche bspw. durch Erhitzen oder elektrochemisch erfolgen kann, werden die verschiedenen Fraktionen der Wasserprobe mittels einer zweiten Verteilstation abwechslungsweise auf eine Leitfähigkeitsmesseinrichtung gelenkt, um die elektrische Leitfähigkeit an mindestens zwei der Fraktionen zu erfassen. Da alle im Wasser gelösten Ionen zur elektrischen Leitfähigkeit beitragen, ermöglicht das Verfahren jene Anteile des Wassers, insbesondere die Konzentrationen der Magnesium- und der Calcium-Ionen, genauer zu bestimmen, welche vorwiegend für die Härte des Wassers bestimmend sind, indem zwei Werte der elektrischen Leitfähigkeit unter verschiedenen Bedingungen gemessen werden. Dabei kann eine kontinuierliche Messung der elektrischen Leitfähigkeit der einzelnen Fraktionen und daher eine kontinuierliche in-situ-Bestimmung der Härte des Wassers erfolgen. Die zur Durchführung dieses Verfahrens zur Bestimmung der Gesamthärte von Wasser eingesetzte Vorrichtung ist allerdings aufwendig, da eine erste und eine zweite Verteilstation zum Trennen der Wasserprobe in mindestens zwei Fraktionen und zum abwechselnden Leiten der Fraktionen auf die Leitfähigkeitsmesseinrichtung benötigt werden.

DE 10 2016 100 195 A1 offenbart ein Verfahren zur Härtebestimmung von Wasser in Wassererhitzungsgeräten, bei dem die Leifähigkeit des verwendeten Wassers zu Beginn eines Aufheizvorgangs und zu einem späteren Zeitpunkt erfasst wird, und die Differenz der Leitfähigkeitswerte als ein Maß für den Härtegrad des Wassers verwendet wird.

Hiervon ausgehend liegt der Erfindung die Aufgabe zu Grunde, ein Verfahren und eine Vorrichtung zur Bestimmung der Härte des Wassers einer Wasserprobe aufzuzeigen, das bei einem einfachen und kostengünstigen Aufbau eine möglichst genaue Erfassung der Gesamthärte des Wassers der Wasserprobe ohne den Einsatz von Chemikalien bei geringen Betriebskosten ermöglicht und dabei wartungsarm und langzeitstabil ist und ohne eine Kalibrierung auskommt. Die Genauigkeit der Bestimmung der Gesamthärte des Wassers soll dabei insbesondere die Vorgaben der Norm DIN 19636-100 "Enthärtungsanlagen (Kationenaustauscher) in der Trinkwasserinstallation" für die Genauigkeit der Verschneidung erfüllen. Weiterhin soll das Verfahren insbesondere eine in-situ-Bestimmung der Gesamthärte eines Wasserstroms gewährleisten, um bspw. in Wasserbehandlungsanlagen, insbesondere in Wasserenthärtungsanlagen oder in Entsalzungsanlagen, eine kontinuierliche Bestimmung der Härte von Rohwasser, das der Wasserbehandlungsanlage zugeführt wird, und/oder der Härte von in der Wasserbehandlungsanlage behandeltem, insbesondere enthärtetem oder entsalztem Wasser, zu ermöglichen.

Diese Aufgaben werden mit dem Verfahren gemäß Anspruch 1 und der Vorrichtung mit den Merkmalen des Anspruchs 10 gelöst. Bevorzugte Ausführungsformen des Verfahrens und der Vorrichtung ergeben sich aus den abhängigen Ansprüchen.

In dem erfindungsgemäßen Verfahren zur Bestimmung der Härte von Wasser einer Wasserprobe, welche Erdalkali-Ionen, insbesondere Calcium- und/oder Magnesium-Ionen, sowie im Überschuss zu den Erdalkali-Ionen Hydrogencarbonat-Ionen enthält, werden folgende Schritte nacheinander ausgeführt:

- Erfassung der ursprünglichen elektrischen Leitfähigkeit ($Lf_1$) der Wasserprobe,
- Fällung der Erdalkali-Ionen durch Elektrolyse der Wasserprobe in einer Elektrolysezelle,
- Erfassung der Leitfähigkeit ($Lf_2$) der Wasserprobe nach einer vollständigen oder zumindest weitgehend vollständigen Fällung der Erdalkali-Ionen und Erfassung des Betrags der durch die Fällung der Erdalkali-Ionen hervorgerufenen Änderung der Leitfähigkeit ($\Delta Lf = |Lf_2 - Lf_1|$) der Wasserprobe,
- Bestimmung der Gesamthärte des Wassers der Wasserprobe aus der erfassten Änderung der Leitfähigkeit ($\Delta Lf$) und einem vorgegebenen Umrechnungsfaktor (F) oder einer vorgegebenen Kennlinie des Verlaufs der Leitfähigkeitsänderung von Wasser in Abhängigkeit der Härteänderung des Wassers bei einer Ausfällung der Carbonathärte.

Das erfindungsgemäße Verfahren geht davon aus, dass in einer Wasserprobe, in der ein Überschuss der Hydrogencarbonat-Ionen gegenüber den Erdalkali-Ionen vorliegt, was bei einigen Trinkwässern von Natur aus der Fall ist, bei einer zumindest weitgehend vollständigen elektrolytischen Ausfällung der Erdalkali-Ionen in Form von Carbonaten, aus der durch die Fällung der Erdalkali-Ionen hervorgerufenen Änderung der Leitfähigkeit ($\Delta Lf = Lf_2 - Lf_1$), insbesondere aus der Erniedrigung der Leitfähigkeit, die Gesamthärte des Wassers der Wasserprobe bestimmt werden kann, weil die Differenz der Leitfähigkeit der Wasserprobe vor und nach der Fällung der Erdalkali-Ionen eine Bestimmung der Gesamtkonzentration der sich im Wasser befindlichen Erdalkali-Ionen, deren Summenkonzentration die Gesamthärte des Wassers definiert, ermöglicht. Bei einem Überschuss der Hydrogencarbonat-Ionen gegenüber den Erdalkali-Ionen, der in dem erfindungsgemäßen Verfahren vorausgesetzt wird, entspricht bei der Elektrolyse der Wasserprobe die Carbonathärte der Gesamthärte des Wassers der sich in der Elektrolysezelle befindlichen Wasserprobe. Die durch die zumindest im Wesentlichen vollständige Fällung der Erdalkali-Ionen hervorgerufene Änderung der Leitfähigkeit stellt daher ein Maß für die Konzentration der härtebildenden Erdalkali-Ionen in der Wasserprobe dar, weshalb aus der erfassten Änderung der Leitfähigkeit ($\Delta Lf$) mittels eines vorgegebenen Umrechnungsfaktors (F) oder einer vorgegebenen Kennlinie, welche den

3

Verlauf der Leitfähigkeitsänderung von Wasser in Abhängigkeit der Härteänderung des Wassers bei einer Ausfällung der Carbonathärte charakterisiert, auf die Gesamthärte des Wassers der Wasserprobe geschlossen werden kann. Die Änderung der Leitfähigkeit ($\Delta$Lf) wird dabei in dem erfindungsgemäßen Verfahren dadurch ermittelt, dass zunächst die ursprüngliche elektrische Leitfähigkeit ($Lf_1$) der Wasserprobe gemessen wird und nach einer zumindest im Wesentlichen vollständigen Fällung der Erdalkali-Ionen in der Elektrolysezelle erneut die elektrische Leitfähigkeit ($Lf_2$) der Wasserprobe gemessen und aus den beiden Messwerten der Betrag der Differenz ($\Delta$Lf = |$Lf_2$ - $Lf_1$|) ermittelt wird.

Die Messung der ursprünglichen elektrischen Leitfähigkeit ($Lf_1$) der Wasserprobe und der elektrischen Leitfähigkeit ($Lf_2$) der Wasserprobe nach der zumindest weitgehend vollständigen Fällung der Erdalkali-Ionen kann dabei mit einem kostengünstigen Leitfähigkeitssensor erfolgen. Die ursprüngliche elektrische Leitfähigkeit ($Lf_1$) der Wasserprobe wird dabei bevorzugt unmittelbar vor Beginn der elektrolytischen Fällung erfasst, kann jedoch auch erst mit oder kurz nach dem Beginn der Fällung ermittelt werden, da die Fällungsreaktion und die daraus resultierende Änderung der Leitfähigkeit erst langsam einsetzt.

Die Elektrolyse der Wasserprobe in der Elektrolysezelle erfolgt in dem erfindungsgemäßen Verfahren zum Zwecke der Fällung der Erdalkali-Ionen. Die weiteren Wirkungen der Elektrolyse der Wasserprobe, insbesondere die Zerlegung des Wassers in Wasserstoff und Sauerstoff, wird dabei nicht ausgenutzt.

Um von der erfassten Änderung der Leitfähigkeit ($\Delta$Lf) durch die zumindest weitgehend vollständige Fällung der Erdalkali-Ionen in der Elektrolysezelle auf die Gesamthärte des Wassers der Wasserprobe schließen zu können, ist es erforderlich, dass die Gesamthärte der Wasserprobe in der Elektrolysezelle einen vollständigen Umsatz bis zum Äquivalenzpunkt der Härtefällung durch eine zumindest weitgehend vollständige Ausfällung der Erdalkali-Ionen in Form von Carbonaten erfährt. Hierfür ist erforderlich, dass in der Wasserprobe ein Überschuss an Hydrogencarbonat-Ionen ($HCO_3^-$) vorhanden ist. Dies ist bei einem Verschnittwasser, das durch Vermischung von Rohwasser mit enthärtetem Wasser, das durch Enthärten des Rohwassers gewonnen wird, zumindest bei üblichen Mischungsverhältnissen automatisch der Fall, weil im Verschnittwasser die Konzentration an Hydrogencarbonat erhalten bleibt und im Vergleich zum Rohwasser die Hydrogencarbonat-Ionen gegenüber den Erdalkali-Ionen im Überschuss vorhanden sind. Um diese Bedingung auch bei einer Wasserprobe sicher einzuhalten, die nicht durch Vermischen von Rohwasser mit enthärtetem Wasser erzeugt worden ist, bspw. bei einem Rohwasser, kann es zweckmäßig sein, die Wasserprobe vor der Messung der Leitfähigkeit so zu konditionieren, dass die Hydrogencarbonat-Ionen ($HCO_3^-$) gegenüber den Erdalkali-Ionen im Überschuss vorhanden sind. Dies kann bspw. durch Vermischung der Wasserprobe mit enthärtetem Wasser, das durch Enthärten des Wassers der Wasserprobe gewonnen wird, und/oder durch Zugabe von Hydrogencarbonat-Ionen und/oder Carbonat-Ionen zur Wasserprobe erfolgen. Wenn das erfindungsgemäße Messverfahren zur Bestimmung der Gesamthärte von Verschnittwasser, das in einer Enthärtungsanlage durch Vermischen von Rohwasser mit enthärtetem Rohwasser erzeugt wird, eingesetzt werden soll, ist die Bedingung des Überschusses von Hydrogencarbonat-Ionen ($HCO_3^-$) gegenüber den Erdalkali-Ionen zumindest dann automatisch erfüllt, wenn die Härte des Verschnittwassers kleiner als 3°dH ist, weshalb sich das erfindungsgemäße Verfahren besonders zur Bestimmung der Gesamthärte von Verschnittwasser aus Enthärtungsanlagen und/oder zur Bestimmung der Gesamthärte des dafür verwendeten Rohwassers eignet. Dabei wird die Wasserprobe als Verschnittwasser durch Vermischen von Rohwasser mit enthärtetem Rohwasser in einem vorgegebenen Mischungsverhältnis erzeugt, wobei aus der erfassten Änderung der Leitfähigkeit ($\Delta$Lf) die Gesamthärte des Verschnittwassers ermittelt und unter Berücksichtigung des Mischungsverhältnisses auch die Gesamthärte des Rohwassers bestimmt werden kann.

Die Bestimmung der Gesamthärte des Wassers der Wasserprobe aus der erfassten Änderung der Leitfähigkeit ($\Delta$Lf) der Wasserprobe erfolgt in dem erfindungsgemäßen Verfahren über einen Umrechnungsfaktor (F), bei dem es sich insbesondere um einen Proportionalitätsfaktor eines linearen Zusammenhangs zwischen der Leitfähigkeitsänderung von Wasser und der Änderung der Gesamthärte oder der Carbonathärte des Wassers bei einer elektrolytischen Fällung der Carbonathärte handelt. Der Umrechnungsfaktor (F) kann auch aus einer Kennlinie entnommen werden, die einen im Regelfall linearen Verlauf der elektrischen Leitfähigkeit in Abhängigkeit von der Carbonathärte des Wassers beschreibt. Der Umrechnungsfaktor (F) ergibt sich dabei aus dem Verhältnis zwischen der Masse an Härtebildnern, die bei der Elektrolyse des Wassers durch Fällung der Erdalkali-Ionen (als Carbonate) entnommenen werden und der daraus folgenden Reduktion der elektrischen Leitfähigkeit der Wasserprobe durch die aufgrund der Fällung der Erdalkali-Ionen verminderten Ionenkonzentration im Wasser. Im Regelfall verläuft die Reduktion der elektrischen Leitfähigkeit der Wasserprobe dabei linear zur ausgefällten Konzentration der Erdalkali-Ionen, wobei der Umrechnungsfaktor (F) die Proportionalitätskonstante dieses linearen Verlaufs ist.

Der Umrechnungsfaktor (F) oder die Kennlinie können dabei experimentell und insbesondere empirisch an einer Vielzahl von unterschiedlichen Wasserproben, insbesondere von verschiedenen Trinkwasserproben unterschiedlicher Herkunft oder Zusammensetzung mit einem Überschuss von Hydrogencarbonat-Ionen gegenüber den Erdalkali-Ionen ermittelt werden, indem an den Wasserproben durch eine Titration der Zusammenhang zwischen der titrimetrisch bestimmten Gesamthärte und der Änderung der elektrischen Leitfähigkeit aufgrund einer vollständigen Ausfällung der Erdalkali-Ionen ermittelt und daraus ein Verlauf der Leitfähigkeitsänderung in Abhängigkeit der Gesamthärte des Wassers in einem Diagramm bzw. einer Kennlinie ermittelt wird. Hierfür kann eine gebräuchliche Härteskala, insbesondere die deutsche Wasserhärte oder die französische Wasserhärte, als Maß für die Gesamthärte verwendet werden. Die Gesamthärte wird bei der Bestimmung des Umrechnungsfaktors oder der Kennlinie titrimetrisch bestimmt, bspw. durch EDTA-Titration (gemäß DIN 38406-3, Gruppe E- Teil 3).

Der Umrechnungsfaktor (F) oder die Kennlinie können auch experimentell an Wasserproben mit einer ausreichenden Konzentration von Erdalkali-Ionen ohne einen vorgegebenen Überschuss von Hydrogencarbonat-Ionen (also insbesondere ohne eine Vorkonditionierung der Wasserprobe) ermittelt werden, indem an den Wasserproben eine insbesondere elektrolytische Fällung der Carbonate durchgeführt wird und dabei sowohl die durch die Fällung hervorgerufene Änderung der elektrischen Leitfähigkeit als auch titrimetrisch die Änderung der Härte (Gesamthärte oder Carbonathärte) der Wasserprobe in einem Diagramm erfasst und daraus der Zusammenhang zwischen der Leitfähigkeitsänderung und der Härteänderung ermittelt wird. Der Umrechnungsfaktor F ergibt sich dabei als Proportionalitätsfaktor aus dem im Regelfall linearen Verlauf der Leitfähigkeitsänderung in Abhängigkeit der Härteänderung.

Unter der Annahme, dass durch die zumindest weitgehend vollständige Fällung der Erdalkali-Ionen in der Elektrolysezelle in dem erfindungsgemäßen Messverfahren die gesamten in der Wasserprobe enthaltenen Härtebildner ausgefällt werden, stellt die erfasste Änderung der Leitfähigkeit ($\Delta$Lf) ein Maß für die Gesamthärte des Wassers der Wasserprobe dar, welches über den Umrechnungsfaktor (F) oder die Kennlinie auf eine gebräuchliche Härteskala zurückgeführt und dadurch als Gesamthärte des Wassers ausgedrückt werden kann. Dabei erfolgt ein Rückschluss von der ausgefällten (temporären) Härte auf die (ursprüngliche) Gesamthärte der Wasserprobe über Massenbilanzrechnungen. Wenn die Randbedingung des Messverfahrens erfüllt ist, wonach die Hydrogencarbonat-Ionen im Überschuss zu den Erdalkali-Ionen in der Wasserprobe, deren Härte bestimmt werden soll, vorhanden sind, entspricht die Gesamthärte der Carbonathärte der Wasserprobe.

Um eine zumindest weitgehend vollständige Fällung der Erdalkali-Ionen in der Elektrolysezelle und damit eine hohe Genauigkeit der bestimmten Gesamthärte des Wassers zu gewährleisten, wird in dem erfindungsgemäßen Verfahren die Leitfähigkeit der Wasserprobe während der elektrolytischen Fällung der Erdalkali-Ionen bevorzugt bis zu einem Umschlagspunkt (U) erfasst, bei dem die erfasste Leitfähigkeit aufgrund einer vollständigen Fällung der Erdalkali-Ionen einen Minimalwert ($Lf_2$) aufweist. Bei einer weiteren Elektrolyse der Wasserprobe über den Umschlagspunkt (U) hinaus, ist ein Anstieg der erfassten Leitfähigkeit der Wasserprobe zu beobachten, der sprunghaft oder graduell sein kann. Dieser Anstieg der Leitfähigkeit ermöglicht eine eindeutige Bestimmung des Minimalwerts ($Lf_2$) der Leitfähigkeit aus dem zeitlichen Verlauf der während der elektrochemischen Fällung gemessenen Leitfähigkeit der Wasserprobe und damit eine genaue Bestimmung der durch eine vollständige Fällung der Erdalkali-Ionen hervorgerufenen Änderung der Leitfähigkeit ($\Delta$Lf).

Um eine ausreichende Genauigkeit der bestimmten Gesamthärte des Wassers der Wasserprobe zu erzielen, die insbesondere den Vorgaben der Norm DIN 19636-100 entspricht, wird die Leitfähigkeit der Wasserprobe während der elektrolytischen Fällung der Erdalkali-Ionen bevorzugt zumindest solange erfasst, bis mindestens 90% der Erdalkali-Ionen, besonders bevorzugt mehr als 95%, durch die Elektrolyse ausgefällt worden sind.

Zur Fällung der Erdalkali-Ionen wird die Wasserprobe bevorzugt in eine Elektrolysezelle eingebracht, in der mindestens zwei Elektrolyseelektroden angeordnet sind und zur elektrolytischen Fällung der Erdalkali-Ionen mit Gleichstrom beaufschlagt werden. Die Elektrolyseelektroden können dabei bspw. als im Abstand und parallel zueinander angeordnete leitfähige Platten (Flachelektroden) ausgebildet sein. Die Wasserprobe kann dabei zwischen den beiden Elektrolyseelektroden im Durchflussbetrieb kontinuierlich durch die Elektrolysezelle geleitet oder in einem Batchbetrieb zwischen die beiden Elektrolyseelektroden in die dann abgeschlossene Elektrolysezelle eingebracht werden.

Zur Erfassung der Leitfähigkeit der Wasserprobe vor und/oder während sowie nach der Fällung der Erdalkali-Ionen enthält die Elektrolysezelle zweckmäßig wenigstens einen Leitfähigkeitssensor, der bevorzugt in die Elektrolysezelle integriert ist. Dies ermöglicht einen kompakten Aufbau. Der in die Elektrolysezelle integrierte Leitfähigkeitssensor umfasst dabei bevorzugt zwei Messelektroden, welche zur Erfassung der Leitfähigkeit der Wasserprobe mit einer Wechselspannung beaufschlagt werden.

Zur Vermeidung von Störeinflüssen ist es dabei vorteilhaft, wenn in der Elektrolysezelle in einem Messmodus zeitlich abwechselnd eine elektrolytische Fällung der Erdalkali-Ionen und eine Messung der Leitfähigkeit der Wasserprobe erfolgt. Dies entkoppelt die elektrolytische Fällung der Erdalkali-Ionen und die Messung der Leitfähigkeit mit dem wenigstens einen in die Elektrolysezelle integrierten Leitfähigkeitssensor.

In einer bevorzugtes Ausführungsform des erfindungsgemäßen Verfahrens wird in dem Messmodus ein bestimmtes Volumen einer Wasserprobe, welches zweckmäßig dem Innenvolumen der Elektrolysezelle entspricht, im Batchbetrieb (Chargenbetrieb) in die Elektrolysezelle eingebracht. Unmittelbar vor Beginn der elektrolytischen Fällung der Erdalkali-Ionen wird die ursprüngliche Leitfähigkeit $Lf_1$ der Wasserprobe in einem ersten Messzyklus erfasst. Danach kann die elektrolytische Fällung der Erdalkali-Ionen beginnen, indem während eines ersten Elektrolysezyklus eine Gleichspannung an die Elektrolyseelektroden angelegt wird. Dabei wird der in die Elektrolysezelle integrierte Leitfähigkeitssensor während des ersten Elektrolysezyklus abgestellt, d.h. die Messelektroden werden während der elektrolytischen Fällung der Erdalkali-Ionen nicht mit Wechselspannung beaufschlagt. An den ersten Elektrolysezyklus können sich dann abwechselnd weitere Messzyklen und weitere Elektrolysezyklen anschließen, um die im Wasser enthaltenen Erdalkali-Ionen auszufällen und während der Fällung punktuell oder quasi-kontinuierlich den Verlauf der Leitfähigkeit der Wasserprobe zu erfassen, bis die Erdalkali-Ionen zumindest im Wesentlichen vollständig ausgefällt sind. Nach der zumindest weitgehend vollständigen Fällung der Erdalkali-Ionen werden die Elektrolyseelektroden stromlos geschaltet und es wird in einem letzten Messzyklus die Leitfähigkeit $Lf_2$ der Wasserprobe erfasst und durch Differenzbildung der ursprünglichen Leitfähigkeit $LF_1$ und der im letzten Messzyklus gemessenen Leitfähigkeit $Lf_2$ die durch die Fällung der Erdalkali-Ionen hervorgerufene Änderung der Leitfähigkeit $\Delta Lf = |Lf_2 - Lf_1|$ ermittelt. Die Dauer der Elektrolysezyklen liegt dabei bevorzugt zwischen 2 Minuten und 10 Minuten und beträgt bspw. 5 Minuten und die Dauer der Messzyklen beträgt zweckmäßig zwischen 30 Sekunden und 120 Sekunden und beträgt bspw. 60 Sekunden.

Im Messmodus wird während der Elektrolysezyklen eine Gleichspannung an die Elektrolyseelektroden mit einer ersten Polarität angelegt, um das Wasser der Wasserprobe in der Elektrolysezelle zu elektrolysieren und dabei die Erdalkali-Ionen auszufällen. Während der Elektrolysezyklen scheiden sich die ausgefällten Carbonate, insbesondere Calcium- und Magnesium-Carbonat, an den Elektrolyseelektroden (insbesondere der Kathode) ab, was zu einer Verfälschung der Messergebnisse führen kann, wenn die Dicke der Abscheidungen zu groß wird. Daher wird bevorzugt in einem Regeneriermodus, der insbesondere nach einer bestimmten Betriebsdauer der Vorrichtung und/oder einer bestimmten Anzahl an durchgeführten Messzyklen oder zu bestimmten Zeiten eingeleitet wird, eine Gleichspannung an die Elektrolyseelektroden mit einer zur ersten Polarität entgegengesetzten Polarität angelegt, um die Carbonatabscheidungen auf der Kathode zu entfernen.

Um den Einfluss der Temperatur auf die Leitfähigkeit der Wasserprobe zu berücksichtigen, wird bevorzugt im Messmodus und insbesondere während der Messzyklen die Temperatur der Wasserprobe gemessen und bei der rechnerischen Bestimmung der Gesamthärte des Wassers der Wasserprobe aus der erfassten Änderung der Leitfähigkeit ($|\Delta Lf|$) erfolgt bevorzugt eine Temperaturkorrektur über einen Temperaturkorrekturfaktor oder eine Temperaturkennlinie, welche die Abhängigkeit der Leitfähigkeit von Wasser von der Temperatur darstellt. Bevorzugt umfasst die Elektrolysezelle hierfür einen Temperatursensor zur Erfassung der Temperatur der Wasserprobe. Der Temperatursensor kann zweckmäßig auch in dem Leitfähigkeitssensor integriert sein.

Die erfindungsgemäße Vorrichtung zur Erfassung der Härte von Wasser einer Wasserprobe dient insbesondere zur Durchführung des oben beschriebenen Verfahrens und umfasst eine Elektrolysezelle mit wenigstens zwei Elektrolyseelektroden und mindestens einen Leitfähigkeitssensor, der bevorzugt in die Elektrolysezelle integriert ist, sowie eine Steuer- und Auswerteeinrichtung, welche so eingerichtet ist, dass sie zunächst die ursprüngliche elektrische Leitfähigkeit ($Lf_1$) der Wasserprobe erfasst, danach eine Gleichspannung an die Elektrolyseelektroden (A, K) mit einer ersten Polarität anlegt, um die Erdalkali-Ionen der Wasserprobe zumindest teilweise und bevorzugt vollständig auszufällen und schließlich die Leitfähigkeit ($Lf_2$) der Wasserprobe während und/oder nach der Fällung der Erdalkali-Ionen erfasst und die Differenz der ursprünglichen Leitfähigkeit und der nach der Fällung der Erdalkali-Ionen gemessenen Leitfähigkeit ($\Delta Lf = |Lf_2 - Lf_1|$) ermittelt.

Zur Bestimmung der Härte des Wassers der Wasserprobe enthält die Steuer- und Auswerteeinrichtung zweckmäßig einen Datenspeicher, in dem der Umrechnungsfaktor (F) oder die Kennlinie des Verlaufs der Leitfähigkeit von Wasser in Abhängigkeit der Carbonathärte des Wassers gespeichert ist. Die Steuer- und Auswerteeinrichtung greift dabei auf den Umrechnungsfaktor (F) oder die Kennlinie zu und ist bevorzugt so eingerichtet, dass sie aus der erfassten Änderung der Leitfähigkeit ($\Delta Lf$) und dem Umrechnungsfaktor (F) oder der Kennlinie die Gesamthärte des Wassers der Wasserprobe auf einer dem Umrechnungsfaktor (F) oder der Kennlinie zugeordneten Härteskala berechnet.

Die Elektrolyseelektroden, welche zur elektrolytischen Fällung der Erdalkali-Ionen an eine Gleichstromquelle angeschlossen werden, sind dabei bevorzugt in der Elektrolysezelle einander gegenüberliegend angeordnet und zweckmäßig als Flachelektroden ausgebildet. Dadurch kann ein ausreichendes Volumen der Elektrolysezelle zur Aufnahme der Wasserprobe bei einem kompakten Aufbau der Vorrichtung bereitgestellt werden. Die Elektrolyseelektroden umfassen mindestens eine Anode, die bevorzugt aus platiniertem Titanblech oder einem platinierten Titangitter oder aus einem Graphitfilm gefertigt ist, und mindestens eine Kathode, die bevorzugt aus Stahl oder Titan oder Graphitfolie gefertigt ist. Besonders bevorzugt ist die Anode und die Kathode aus demselben Material, insbesondere jeweils aus platiniertem Titan.

Zur Erfassung der Leitfähigkeit der Wasserprobe vor und/oder während sowie nach der Fällung der Erdalkali-Ionen umfasst die Vorrichtung wenigsten einen Leitfähigkeitssensor. Bevorzugt ist wenigstens ein Leitfähigkeitssensor in der Elektrolysezelle integriert, wobei dieser Leitfähigkeitssensor mindestens ein Elektrodenpaar mit zwei Messelektroden, die an eine Wechselspannungsquelle angeschlossen oder anschließbar sind, umfasst. Die Integration des einen Leitfähigkeitssensors oder der mehreren Leitfähigkeitssensoren in die Elektrolysezelle ermöglicht einen kompakten und platzsparenden Aufbau der Vorrichtung. Es ist jedoch auch möglich, dass zumindest ein erster Leitfähigkeitssensor und ein zweiter Leitfähigkeitssensor außerhalb oder auch innerhalb der Elektrolysezelle angeordnet ist. So kann bspw. ein erster Leitfähigkeitssensor an einem Eingang, insbesondere stromaufwärts des Eingangs der Elektrolysezelle und ein zweiter Leitfähigkeitssensor an einem Ausgang, insbesondere stromabwärts des Ausgangs der Elektrolysezelle, platziert sein.

Zur Erfassung der elektrischen Leitfähigkeit können auch andere Leitfähigkeitssensoren eingesetzt werden, wie z.B. Hochfrequenztitrationssensoren.

In einer bevorzugten Ausführungsform der Vorrichtung, die insbesondere für eine Durchführung des oben beschrieben Verfahrens im Batchbetrieb geeignet ist, umfasst die Vorrichtung einen einzigen, in die Elektrolysezelle integrierten Leitfähigkeitssensor mit zwei Messelektroden, die zwischen den einander gegenüberliegenden Elektrolyseelektroden der Elektrolysezelle angeordnet und insbesondere als Stabelektroden ausgebildet sind. Dies ermöglicht eine kostengünstige Herstellung der Vorrichtung in einer kompakten Bauweise.

In einer Ausführungsform der erfindungsgemäßen Vorrichtung kann die Wasserprobe im Messmodus als Wasserstrom kontinuierlich im Durchflussbetrieb durch die Elektrolysezelle strömen. Das Wasser strömt dabei insbesondere zwischen den beiden gegenüberliegenden Elektrolyseelektroden von einem Eingang bis zu einem Ausgang durch die Elektrolysezelle. Dabei wird die ursprüngliche Leitfähigkeit $Lf_1$ der Wasserprobe stromaufwärts der Elektrolysezelle oder an einem Eingang der Elektrolysezelle mit einem ersten Leitfähigkeitssensor erfasst. Dies kann kontinuierlich erfolgen, insbesondere mit einem außerhalb der Elektrolysezelle oder am Eingang der Elektrolysezelle angeordneten ersten Leitfähigkeitssensor, oder in vorgegebenen Messzyklen insbesondere mit einem innerhalb der Elektrolysezelle angeordneten ersten Leitfähigkeitssensor. Beim Durchlauf des Wasserstroms vom Eingang der Elektrolysezelle bis zum Ausgang werden die Erdalkali-Ionen zumindest teilweise, bevorzugt vollständig ausgefällt. Am Ausgang der Elektrolysezelle oder stromabwärts der Elektrolysezelle ist dabei ein zweiter Leitfähigkeitssensor angeordnet, mit dem die Leitfähigkeit $Lf_2$ des Wasserstroms gemessen wird. Aus der Differenz der am Eingang und am Ausgang der Elektrolysezelle gemessenen Leitfähigkeiten wird die durch das Ausfällen der Erdalkali-Ionen hervorgerufene Änderung der Leitfähigkeit $\Delta Lf = |Lf_2 - Lf_1|$ ermittelt. Wenn die so ermittelte Änderung der Leitfähigkeit $\Delta Lf$ über einem vorgegebenen Grenzwert liegt, kann darauf geschlossen werden, dass in dem Wasserstrom, der am Eingang in die Elektrolysezelle einströmt, härtebildende Erdalkali-Ionen vorhanden sind, die beim Durchströmen durch die Elektrolysezelle zumindest teilweise ausgefällt worden sind, wodurch sich die erfasste Änderung der Leitfähigkeit $\Delta Lf$ ergibt. Mit dieser Ausführungsform der erfindungsgemäßen Vorrichtung lassen sich bspw. in Enthärtungsanlagen Härtedurchbrüche erfassen, die bei einer Erschöpfung des oder der Ionenaustauscher auftreten können.

Zur Beschleunigung der Ausfällung der Erdalkali-Ionen in den Messzyklen kann zwischen den zwei gegenüberliegenden Elektrolyseelektroden der Elektrolysezelle eine Kationenaustauschermembran angeordnet sein, wobei die Kationenaustauschermembran insbesondere parallel zu den als Flachelektroden ausgebildeten Elektrolyseelektroden verläuft. Weiterhin können in der Elektrolysezelle auch mehrere Paare von Elektrolyseelektroden im Abstand zueinander angeordnet sein, insbesondere in Form von Flachelektroden, die parallel zueinander verlaufen. So kann bspw. ein Kaskaden-Anordnung von korrespondierenden Elektrolyseelektroden in der Reihenfolge Anode - Kathode - Anode - Kathode - Anode in der Elektrolysezelle vorgesehen sein. Dies beschleunigt ebenfalls die Ausfällung der Erdalkali-Ionen in den Messzyklen und reduziert daher die Messdauer, d.h. die Dauer bis zur vollständigen Ausfällung der Erdalkali-Ionen.

Die erfindungsgemäße Vorrichtung eignet sich insbesondere zur Verwendung in einer Wasserbehandlungsanlage, wie z.B. in einer Wasserenthärtungsanlage oder einer Entsalzungsanlage und insbesondere in einer Umkehrosmoseanlage (RO) oder einer membrankapazitiven Deionisationsanlage (MCDI) oder einer Nanofiltrationsanlage (NF), um die Härte von Rohwasser, das der Wasserbehandlungsanlage zugeführt wird, und/oder die Härte von in der Wasserbehandlungsanlage behandeltem, insbesondere enthärtetem oder entsalztem Wasser, zu bestimmen. Die Vorrichtung kann auch in Wasserenthärtungsanlagen oder in Entsalzungsanlagen eingesetzt werden, um Härtedurchbrüche zu detektieren, die bspw. bei einer Erschöpfung von Ionenaustauschern oder durch Verblocken einer (Filter- oder Osmose-) Membran auftreten können. Die erfindungsgemäße Vorrichtung kann dabei in der Wasserbehandlungsanlage integriert sein, um während des Betriebs der Wasserbehandlungsanlage eine ins-situ-Bestimmung der Härte des Rohwassers und/oder des behandelten Wassers vorzunehmen. Es ist jedoch auch möglich, die Vorrichtung als mobiles Messgerät auszubilden, mit dem bspw. bei einer Erstinbetriebnahme einer Wasserbehandlungsanlage eine Einstellung der Betriebsparameter unter Bestimmung der Härte des Rohwassers und/oder des behandelten Wassers vorgenommen werden kann. Weitere Anwendungen des Verfahrens und der Vorrichtung sind bspw. die Bestimmung der Härte von Wasser, das einer Waschmaschine oder einer Spülmaschine zugeführt wird, um basierend auf der bestimmten Härte des zugeführten Wassers eine Dosierung eines Wasch- oder Spülmittels und/oder eines Entkalkungsmittels einzustellen oder automatisiert vorzunehmen.

Mit dem erfindungsgemäßen Verfahren und der erfindungsgemäßen Vorrichtung kann die Gesamthärte des Wassers einer Wasserprobe, deren Gesamthärte der Carbonathärte (temporäre Härte) entspricht, mit für die oben genannten Anwendungen ausreichender Genauigkeit bestimmt werden. Dabei wird, anders als bspw. bei einer thermischen Ausfällung, durch die elektrolytische Fällung der Erdalkali-Ionen, die gesamte Abscheidekapazität der Erdalkali-Ionen ausgenutzt, wodurch die gesamte Carbonathärte und damit zumindest näherungsweise (und zumindest für die meisten Trinkwasserqualitäten) die Gesamthärte des Wassers der Wasserprobe bestimmt werden kann. Das Verfahren und die Vorrichtung ermöglichen dabei eine in-situ-Erfassung der Änderung der Leitfähigkeit, die durch die Fällung der Erdalkali-Ionen hervorgerufen wird, und darauf basierend eine in-situ-Bestimmung der Härte des Wassers der Wasserprobe in einem automatisierten Verfahren ohne Verwendung von Chemikalien.

Diese und weitere Vorteile und Anwendungen sowie bevorzugte Merkmale des erfindungsgemäßen Verfahrens und der erfindungsgemäßen Vorrichtung ergeben sich aus den nachfolgend unter Bezugnahme auf die Zeichnungen beschriebenen Ausführungsbeispiele der Erfindung.

Dabei zeigen:

Fig. 1:    eine schematische Darstellung einer ersten Ausführungsform einer erfindungsgemäßen Vorrichtung zur Bestimmung der Härte von Wasser einer Wasserprobe;

Fig. 2:    eine schematische Darstellung einer zweiten Ausführungsform einer erfindungsgemäßen Vorrichtung;

Fig. 3:    eine schematische Darstellung einer dritten Ausführungsform einer erfindungsgemäßen Vorrichtung;

Fig. 4:    eine schematische Darstellung eines Anwendungsbeispiels für das erfindungsgemäße Verfahren und die erfindungsgemäße Vorrichtung zur Bestimmung der Härte von Wasser, welches als Verschnittwasser in einer Wasserenthärtungsanlage erzeugt worden ist;

Fig. 5:    eine schematische Darstellung eines weiteren Anwendungsbeispiels des erfindungsgemäßen Verfahrens und der erfindungsgemäßen Vorrichtung zur Bestimmung der Härte von in einer Wasserbehandlungsanlage behandeltem Wasser;

Fig. 6:    ein Diagramm eines typischen zeitlichen Verlaufs der elektrischen Leitfähigkeit einer Wasserprobe bei der Durchführung des erfindungsgemäßen Verfahrens;

Fig. 7:    ein Diagramm des zeitlichen Verlaufs der in einem Anwendungsfall des erfindungsgemäßen Verfahrens erfassten elektrischen Leitfähigkeit einer Wasserprobe sowie der mit dem erfindungsgemäßen Verfahren bestimmten Härte der Wasserprobe.

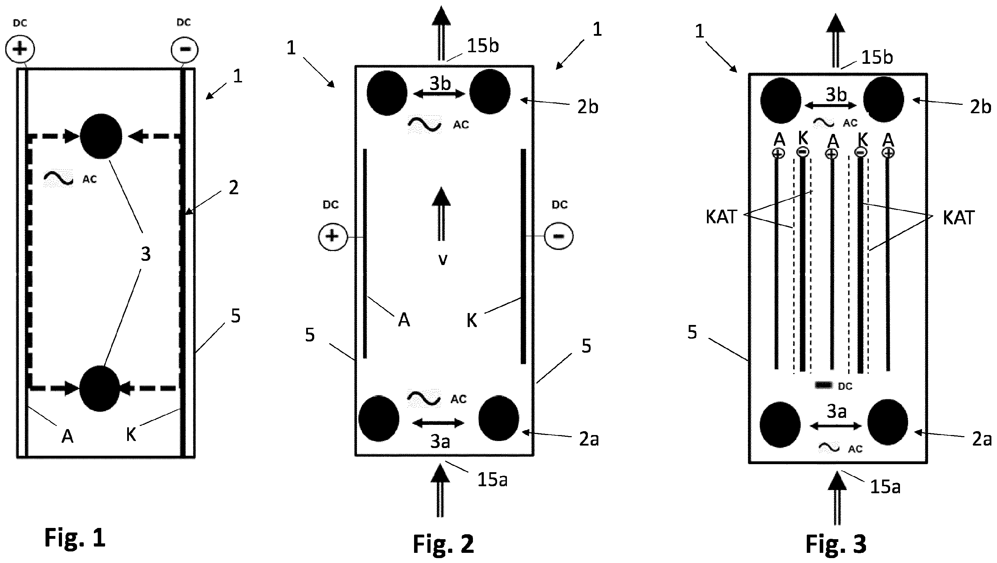

In Figur 1 ist schematisch eine erste Ausführungsform einer erfindungsgemäßen Vorrichtung zur Bestimmung der Härte von Wasser einer Wasserprobe gezeigt. Die Vorrichtung der Figur 1 umfasst eine Elektrolysezelle 1 mit einem Behälter 5, der über einen hier nicht dargestellten Zugang zum Einbringen der Wasserprobe und zum Ablassen der Wasserprobe nach erfolgter Härtebestimmung enthält. Der Zugang ist verschließbar, so dass sich das Wasser nach Einbringen der Wasserprobe in den Behälter 5 in einem abgeschlossenen Volumen befindet. Die Elektrolysezelle 1 wird in dieser Ausführungsform zur Bestimmung der Härte des Wassers der Wasserprobe im Batchbetrieb (Chargenbetrieb) verwendet, in dem die Härte des Wassers eines vorgebebenen Volumens einer Wasserprobe bestimmt wird.

Innerhalb des Behälters 5 sind zwei im Abstand und parallel zueinander verlaufende Flachelektroden angeordnet. Die beiden Flachelektroden bilden einander zugeordnete Elektrolyseelektroden A, K, wobei die eine Flachelektrode eine Anode (A) und die andere Flachelektrode eine Kathode (K) bildet. Die beiden Elektrolyseelektroden A, K, sind an eine Gleichstromquelle DC angeschlossen, wie in Figur 1 angedeutet. Über die Gleichstromquelle DC werden die beiden Elektrolyseelektroden A, K mit elektrischem Gleichstrom beaufschlagt, Durch die Beaufschlagung der beiden Elektrolyseelektroden A, K mit elektrischem Gleichstrom wird das sich im Behälter 5 der Elektrolysezelle 1 befindliche Wasser der Wasserprobe elektrolysiert, wobei sich die im Wasser befindlichen Erdalkali-Ionen als Carbonate an der Kathode (K) abscheiden und dadurch aus dem Wasser ausgefällt werden.

Zwischen den beiden Elektrolyseelektroden A, K ist ein Leitfähigkeitssensor 2 mit einem Elektrodenpaar 3 angeordnet. Das Elektrodenpaar 3 ist an eine Wechselspannungsquelle AC angeschlossen und kann über die Wechselspannungsquelle AC mit einer elektrischen Wechselspannung mit einer vorgegebenen Frequenz beaufschlagt werden. Das Elektrodenpaar 3 wirkt dabei als Leitfähigkeitssensor 2, mit dem die Leitfähigkeit des sich im Behälter 5 befindlichen Wassers erfasst werden kann. Die beiden Messelektroden des Elektrodenpaars 3 sind in dem in Figur 1 gezeigten Beispiel als Stabelektroden ausgebildet, deren Längsachse parallel zu der Ebene der beiden als Flachelektroden ausgebildeten Elektrolyseelektroden A, K verläuft.

Die in Figur 1 gezeigte Elektrolysezelle 1 mit den beiden Elektrolyseelektroden A, K und dem in der Elektrolysezelle 1 integrierten Leitfähigkeitssensor 2 ist mit einer hier nicht zeichnerisch dargestellten Steuer- und Regeleinrichtung gekoppelt. Die Steuer- und Regeleinrichtung steuert die Versorgung der Elektrolyseelektroden A, K während vorgegebener Elektrolysezyklen mit elektrischem Gleichstrom und die Erfassung der elektrischen Leitfähigkeit des sich im Behälter 5 befindlichen Wassers mittels des Leitfähigkeitssensors 2, indem an das Elektrodenpaar 3 des Leitfähigkeitssensors 2 in definierten Messzyklen eine Wechselspannung angelegt wird. Bevorzugt ist die Steuer- und Regeleinrichtung hierfür so eingerichtet, dass abwechselnd nacheinander Messzyklen mit einer vorgegebenen Messdauer und Elektrolysezyklen mit einer vorgegebenen Elektrolysedauer durchgeführt werden, wobei bevorzugt während eines Messzyklus an das Elektrodenpaar 3 des Leitfähigkeitssensors 2 eine elektrische Wechselspannung angelegt wird, während die beiden Elektrolyseelektroden A, K stromlos geschaltet sind und während eines Elektrolysezyklus die beiden Elektrolyseelektroden A, K mit elektrischem Gleichstrom beaufschlagt werden, während das Elektrodenpaar 3 spannungslos ist.

Die Steuer- und Regeleinrichtung umfasst einen Datenspeicher und eine Recheneinheit. In dem Datenspeicher ist mindestens eine Kennlinie hinterlegt, die den Verlauf der elektrischen Leitfähigkeit von Wasser in Abhängigkeit der Carbonathärte des Wassers auf einer Härteskala darstellt. Der Datenspeicher der Steuer- und Regeleinrichtung kann auch mehrere solcher Kennlinien enthalten, wobei die einzelnen Kennlinien beispielsweise den Verlauf der elektrischen Leitfähigkeit von Wasser in Abhängigkeit von der Carbonathärte des Wassers auf unterschiedlichen Härteskalen, beispielsweise der Härteskala deutscher Härte (°dH) oder der französischen Härte (°fH) darstellt. Da die Kennlinie einen linearen Verlauf der elektrischen Leitfähigkeit in Abhängigkeit der Carbonathärte des Wassers hat, kann auch der Proportionalitätsfaktor, der die Steigung dieses linearen Verlaufs angibt, als Umrechnungsfaktor F ergänzend oder anstelle der Kennlinie in dem Speicher der Steuer- und Regeleinrichtung abgespeichert sein. Eine Methode zur Bestimmung der Kennlinie und des Umrechnungsfaktors F wird im Folgenden noch erläutert.

Zur Bestimmung der Härte des Wassers der sich im Behälter 5 der Elektrolysezelle 1 befindlichen Wasserprobe wird in einem Messmodus zunächst bei stromlos geschalteten Elektrolyseelektroden A, K eine elektrische Wechselspannung mit einer vorgegebenen Frequenz an das Elektrodenpaar 3 des Leitfähigkeitssensors 2 angelegt und dieStromstärke, des durch die Messelektroden des Elektrodenpaars 3 geleiteten Stroms gemessen, um die ursprüngliche Leitfähigkeit $Lf_1$ des Wassers zu messen. Diese Messung stellt einen ersten Messzyklus dar. Danach wird die Spannungszufuhr der Wechselspannung zu dem Elektrodenpaar 3 des Leitfähigkeitssensors 2 abgestellt und es beginnt ein erster Elektrolysezyklus für eine vorgegebene Elektrolysedauer. Hierfür wird eine Gleichspannung an die beiden Elektrolyseelektroden A, K angelegt. Durch das Anlegen einer Gleichspannung an die beiden Elektrolyseelektroden A, K wird das Wasser in dem Behälter 5 elektrolysiert und gleichzeitig beginnen die im Wasser befindlichen Erdalkali-Ionen als Carbonate, insbesondere als Calcium-Carbonat und als Magnesium-Carbonat auszufällen. Nach Beendigung des ersten Elektrolysezyklus wird die Stromzufuhr zu den Elektrolyseelektroden A, K abgestellt und es erfolgt ein zweier Messzyklus durch Anlegen einer Wechselspannung an das Elektrodenpaar 3 des Leitfähigkeitssensors 2 während einer vorgegebenen Messdauer. Dabei wird die elektrische Leitfähigkeit des Wassers in dem Behälter 5 erfasst. Aufgrund einer teilweise erfolgten Fällung der Erdalkali-Ionen hat sich die Leitfähigkeit, die in dem zweiten Messzyklus gemessen wird, gegenüber der ursprünglichen Leitfähigkeit des Wassers vermindert, da die ausgefällten Erdalkali-Ionen nicht mehr zu dem Stromfluss in der Elektrolysezelle 1 beitragen. Anschließend werden weitere Elektrolysezyklen und Messzyklen abwechselnd nacheinander durchgeführt.

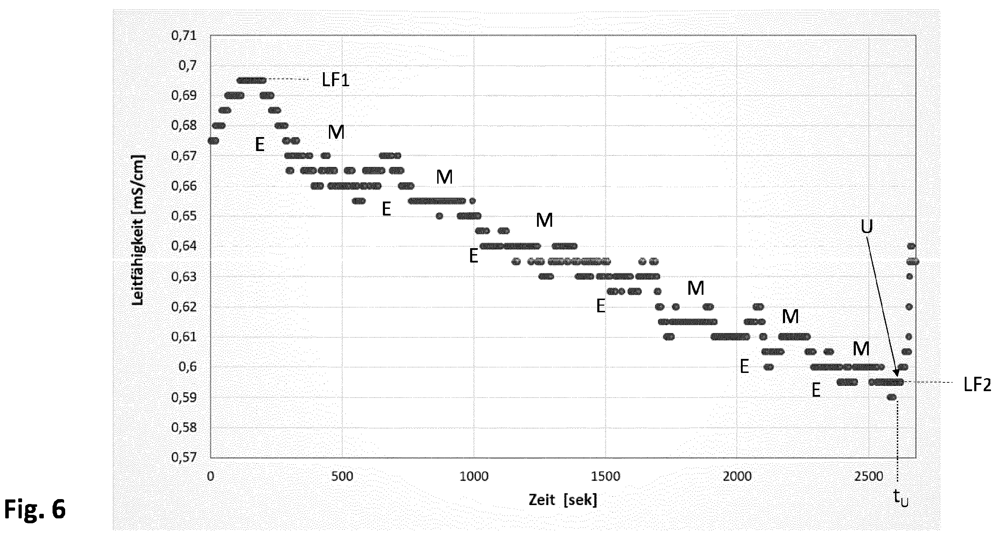

Der sich dadurch ergebende zeitliche Verlauf der elektrischen Leitfähigkeit des sich im Behälter 5 befindlichen Wassers ist anhand eines Beispiels in Figur 6 dargestellt. In dem zeitlichen Verlauf der elektrischen Leitfähigkeit der Figur 6 sind die einzelnen Messzyklen M ersichtlich, in denen sich die Leitfähigkeit des Wassers nicht ändert. Zwischen aufeinander folgenden Messzyklen M mit gleichbleibender Leitfähigkeit befinden sich die Elektrolysezyklen E, wobei die die elektrische Leitfähigkeit des Wassers in jedem Elektrolysezyklus E aufgrund der fortschreitenden Ausfüllung der Erdalkali-Ionen weiter abnimmt, bis zu einem in Figur 6 mit U gekennzeichneten Umschlagspunkt, an dem die mit dem Leitfähigkeitssensor 2 erfasste Leitfähigkeit des Wassers sprunghaft oder graduell ansteigt. Der Anstieg der elektrischen Leitfähigkeit beim Umschlagspunkt U resultiert vermutlich aus einem plötzlichen Anstieg des pH-Werts und einem daraus resultierenden Überschuss von Anionen ($CO_3^{2-}$ und $OH^-$) mit höherer Äquivalentleitfähigkeit gegenüber den Hydrogencarbonat-Ionen nach Abschluss der kathodischen Fällung .

Unter der Annahme, dass während der Elektrolysezyklen alle im Wasser enthaltene Erdalkali-Ionen als Carbonate, insbesondere als Calcium- und Magnesium-Carbonat, ausgefällt worden sind, kann über die Differenz der erfassten Leitfähigkeit vor Beginn der Elektrolyse ($Lf_1$) und der nach vollständiger Ausfällung der Erdalkali-Ionen erfassten Leitfähigkeit ($Lf_2$) die durch die Fällung der Erdalkali-Ionen hervorgerufene Änderung der elektrischen Leitfähigkeit $\Delta Lf = |Lf_2 - Lf_1|$ berechnet werden und aus diesem Differenzwert, welcher der Differenz der ursprünglichen Leitfähigkeit des Wassers und der nach der vollständigen Fällung der Erdalkali-Ionen gemessenen Leitfähigkeit entspricht, kann auf die Gesamthärte bzw. die Carbonathärte des sich im Behälter 5 befindlichen Wassers geschlossen werden. Hierfür ist die Steuer- und Regeleinrichtung so programmiert, dass sie aus den erfassten Messwerten der ursprünglichen Leitfähigkeit $Lf_1$ und der Leitfähigkeit des Wassers nach der vollständigen Fällung der Erdalkali-Ionen $Lf_2$ den Betrag der Differenz dieser Messwerte $\Delta Lf = |Lf_2 - Lf_1|$ berechnet und aus dem Differenzwert unter Rückgriff auf die im Speicher der Steuer- und Regeleinrichtung hinterlegten Kennlinie und/oder des dort hinterlegten Umrechnungsfaktors F die Härte des Wassers auf der Härteskala der Kennlinie bzw. des Umrechnungsfaktors F wie folgt zu bestimmt:

$$H = \Delta Lf / F,$$

wobei H die Gesamthärte des Wassers, $\Delta Lf$ die Differenz der gemessenen Leitfähigkeiten des Wassers vor und nach der vollständigen Fällung der Erdalkali-Ionen und F der Umrechnungsfaktor ist, der sich aus der Kennlinie des (linearen) Verlaufs der Leitfähigkeit von Wasser in Abhängigkeit der Carbonathärte ergibt und insbesondere der Proportionalitätsfaktor eines linearen Verlaufs dieser Kennlinie ist.

Der Umrechnungsfaktor F ergibt sich aus dem Verhältnis zwischen der Masse an entnommenen Härtebildnern durch Fällung und der Reduktion der Leitfähigkeit dank proportional verminderter Ionenkonzentrationen. Bis zum Umschlagspunkt U läuft die Kurve einer elektrolytischen Härtefällung (bei Konstantstrom) nahezu linear, wie aus Figur 6 ersichtlich. Bei Konstantspannung (abnehmende Stromstärke) ist der zeitliche Verlauf der Leitfähigkeit zwar nicht mehr linear, der Umschlagspunkt U liegt jedoch unverändert beim gleichen Wert der Leitfähigkeit (Leitwert). Bei Überschuss an Hydrogencarbonat gegenüber den Erdalkali-Ionen entspricht der Umschlagspunkt U wegen der vergleichbaren Äquivalent-Leitwerte von Ca und Mg recht genau den vorhandenen Summenkonzentrationen an Erdalkali-Ionen.

Der Umrechnungsfaktor F kann experimentell an einer Trinkwasserprobe mit einer ausreichend hohen Wasserhärte von bevorzugt mehr als 3°dH und einem Überschuss an Hydrogencarbonationen gegenüber den Erdalkali-Ionen mit folgenden Schritten ermittelt werden:

1. Messung der ursprünglichen Leitfähigkeit (Leitwert $Lf_A$) sowie der Temperatur (T) der Trinkwasserprobe und Bestimmung der Leitfähigkeit $Lf_A$ ($T_0$) der Trinkwasserprobe bei einer Standardtemperatur $T_0$ von bspw. 15°C mittels eines Temperaturkorrekturfaktors;

2. Durchführen einer Wasseranalyse zur Bestimmung der Gesamthärte H der Trinkwasserprobe auf einer vorgegebenen Härteskala, bspw. der deutschen Härte [°dH], mittels komplexometrischer Titration, bspw. mit Ethylendiamintetraacetat (EDTA) gemäß DIN 38406-3, Teil E3;

3. Elektrolyse der Trinkwasserprobe mit dem Anfangsleitwert $Lf_A$ ($T_0$) unter definierten Randbedingungen, insbesondere der Temperatur, und Erfassung der Leitfähigkeit der Trinkwasserprobe während der Elektrolyse zur Ermittlung einer Verlaufskurve des Leitwerts über die Zeit, wobei der Leitwert linear mit der Zeit abnimmt und sich zu einem bestimmten Zeitpunkt $t_U$ an einem Umschlagspunkt U ein Minimum der Leitfähigkeit (Leitwertminimum $Lf_{min}$) einstellt (s. Figur 6), welches erfasst und mittels des Temperaturkorrekturfaktors auf die Leitfähigkeit bei der Standardtemperatur $T_0$ umgerechnet wird, um das Leitwertminimum $Lf_{min}(T_0)$ bei der Standardtemperatur $T_0$ zu erhalten;

4. Optional nochmalige Elektrolyse unter gleichen Randbedingungen bei nun aus Schritt 3 bekanntem Zeitpunkt $t_U$ des Umschlagspunkts U zur Verifizierung der Verlaufskurve und Verbesserung der Genauigkeit des ermittelten Leitwertminimums $Lf_{min}$ sowie Beendigung der Elektrolyse nach Erreichen des Umschlagspunkts U;

5. Entnahme eines Teilvolumens der Trinkwasserprobe nach Erreichen des Umschlagspunkts U und Durchführen einer titrimetrischen Wasseranalyse der entnommenen Probe zur Bestimmung der Gesamthärte der Trinkwasserprobe nach der Elektrolyse, wobei die titrimetrische Wasseranalyse bevorzugt mit derselben Methode wie in Schritt 2 erfolgt und dabei eine Härte von 0 erfasst wird, wenn in Schritt 3 eine zumindest weitgehend vollständige Ausfällung der Erdalkali-Ionen (nach Erreichen des Umschlagspunkts U) erfolgt ist;

6. Berechnung des Umrechnungsfaktors F aus der Differenz der Leitfähigkeit

$$\text{(Leitwertdifferenz) } \Delta Lf\,(T_0) = Lf_A(T_0) - Lf_{min}\,(T_0) \text{ mit der Formel } F = \Delta Lf\,(T_0)\,/\,H.$$

Die Randbedingung, wonach in der Trinkwasserprobe ein Überschuss an Hydrogencarbonat-Ionen gegenüber den Erdalkali-Ionen vorhanden ist, kann durch eine Konditionierung der Trinkwasserprobe vor Beginn der Bestimmung des Umrechnungsfaktors eingehalten werden, bspw. indem der Trinkwasserprobe Hydrogencarbonat-Ionen zugegeben werden oder indem die Trinkwasserprobe teilenthärtet wird. Dadurch entspricht die Gesamthärte der konditionierten Trinkwasserprobe der Carbonathärte. Alternativ zu einer Konditionierung der Trinkwasserprobe kann auch eine nicht speziell auf einen Überschuss an Hydrogencarbonat-Ionen gegenüber den Erdalkali-Ionen vorkonditionierte Trinkwasserprobe verwendet werden, wenn in den Schritten 2 und 5 die Änderung der Carbonathärte $\Delta H_C$ der Trinkwasserprobe ermittelt wird, die sich durch das Ausfällen der Carbonate ergibt, und der Umrechnungsfaktor aus der durch das Ausfällen hervorgerufenen Änderung der Leitfähigkeit ($\Delta Lf$) und der Änderung der Carbonathärte ($\Delta H_C$) aus der Formel $F = \Delta Lf\,(T_0)\,/\,\Delta H_C$ ermittelt wird.

Mit dieser Methode wurde aus empirischen Messungen an einer Vielzahl von Trinkwasserproben aus Deutschland unterschiedlicher Herkunft und unterschiedlicher Zusammensetzung für den Umrechnungsfaktor F bei einer Temperatur von $T_0 = 15°C$ bspw. ein gemittelter Wert von

$$F = 30\ \mu S/\,(cm\ °dH) = 0{,}030\ mS/\,(cm\ °dH)$$

ermittelt.

Aus dem in Figur 6 gezeigten zeitlichen Verlauf der elektrischen Leitfähigkeit einer Wasserprobe ergibt sich für die Härte des Wassers dieser Wasserprobe daher folgende Berechnung:

$$H = \Delta \mathrm{Lf} \,/\, \mathrm{F} \;=\; \frac{(0{,}70 \;-\; 0{,}59)\ \mathrm{mS/cm}}{0{,}030\ \mathrm{mS/\,(cm\ °dH)}} \;=\; 3{,}6\ \mathrm{°dH}$$

In den Figuren 2 und 3 sind zwei weitere Ausführungsformen einer erfindungsgemäßen Vorrichtung mit einer Elektrolysezelle 1 gezeigt, wobei die Elektrolysezelle 1 zwei voneinander getrennte Leitfähigkeitssensoren 2a, 2b enthält. Ein erster Leitfähigkeitssensor 2a ist dabei an einem verschließbaren Eingang 15a der Elektrolysezelle 1 und ein zweiter Leitfähigkeitssensor 2b an einem verschließbaren Ausgang 15b der Elektrolysezelle 1 angeordnet. Jeder Leitfähigkeitssensor 2a, 2b umfasst dabei jeweils ein Elektrodenpaar 3a, 3b mit zwei Messelektroden. Zwischen dem Eingang 15a und dem Ausgang 15b der Elektrolysezelle 1 sind in dem Ausführungsbeispiel von Figur 2 die beiden Elektrolyseelektroden A, K angeordnet, welche, wie im Ausführungsbeispiel von Figur 1, als Flachelektroden ausgebildet und in einem vorgegebenen Abstand einander gegenüberliegend angeordnet sind. Die in Figur 2 gezeigte Ausführungsform der Elektrolysezelle 1 kann zur Erfassung der Härte von Wasser einer Wasserprobe eingesetzt werden, die im Durchflussbetrieb kontinuierlich vom Eingang 15a zum Ausgang 15b durch die Elektrolysezelle 1 strömt, wobei während des Durchströmens der Wasserprobe kontinuierlich und gleichzeitig Messzyklen und Elektrolysezyklen durchlaufen werden. Während die Wasserprobe durch die Elektrolysezelle 1 strömt, werden die Erdalkali-Ionen in dem Wasser der Wasserprobe zumindest teilweise ausgefällt. Während des Durchströmens der Wasserprobe durch die Elektrolysezelle 1 wird die Leitfähigkeit des Wasserstroms am Eingang 15a und am Ausgang 15b kontinuierlich mit den beiden Leitfähigkeitssensoren 2a, 2b gemessen, wobei der erste Leitfähigkeitssensor 2a die ursprüngliche Leitfähigkeit $Lf_1$ der Wasserprobe am Eingang 15a (und damit vor der elektrolytischen Fällung der Erdalkali-Ionen) erfasst und mit dem zweiten Leitfähigkeitssensor 2b die elektrische Leitfähigkeit $Lf_2$ des Wasserstroms am Ausgang 15b der Elektrolysezelle 1 erfasst wird (und damit nach einer Fällung der Erdalkali-Ionen). Die Steuer- und Auswerteeinrichtung 14 berechnet aus den erfassten Messwerten Lf1 und Lf2 den Betrag der Differenz $\Delta Lf = |Lf_2 - Lf_1|$ und bestimmt somit die durch das Ausfällen der Erdalkali-Ionen hervorgerufene Änderung der Leitfähigkeit. Die berechnete Differenz der elektrischen Leitfähigkeit $\Delta Lf$ der Wasserprobe vor und nach der Fällung der Erdalkali-Ionen wird in der Steuer- und Auswerteeinrichtung 14 mit einem vorgegebenen Grenzwert verglichen. Falls die erfasste Differenz der elektrischen Leitfähigkeit $\Delta Lf$ über diesem Grenzwert liegt, kann darauf geschlossen werden, dass sich eine bestimmte Menge an härtebildenden Erdalkali-Ionen in dem Wasser befindet.

Um die Härte des Wassers des Wasserstroms zu bestimmen, kann die Vorrichtung der zweiten Ausführungsform gemäß Figur 2 vom Durchflussbetrieb in einen Batchbetrieb (Chargenbetrieb) umgestellt werden, indem der Eingang 15a und der Ausgang 15b der Elektrolysezelle geschlossen werden. Danach erfolgt die Bestimmung der Härte des Wassers in dem anhand der Ausführungsform der Figur 1 beschriebenen Messmodus.

In dem in Figur 3 gezeigten Ausführungsbeispiel sind in der Elektrolysezelle 1 mehrere Elektrolyseelektroden A, K angeordnet. Insbesondere enthält die Elektrolysezelle 1 des Ausführungsbeispiels von Figur 3 eine Kaskade von Elektrolyseelektroden in der Folge einer äußeren Anode A, einer ersten Kathode K, einer inneren Anode A, einer zweiten Kathode K sowie einer weiteren äußeren Anode A, wie aus Figur 3 ersichtlich. Zwischen korrespondierenden Elektrolyseelektroden A, K ist jeweils eine Kationenaustauschermembran KAT angeordnet. Durch die Mehrzahl von Elektrolyseelektroden A, K sowie der zwischen korrespondierenden Elektrolyseelektroden A, K angeordneten Kationenaustauschermembrane KAT wird die Effizienz der elektrolytischen Fällung der Erdalkali-Ionen durch die Ausfällung in Form von Erdalkali-Carbonate an den Kathoden K (insbesondere aufgrund einer größeren Elektrodenfläche) erhöht, wodurch sich die Messdauer verringert und/oder die Strömungsgeschwindigkeit, mit der die Wasserprobe durch die Elektrolysezelle 1 strömt, erhöht werden kann.

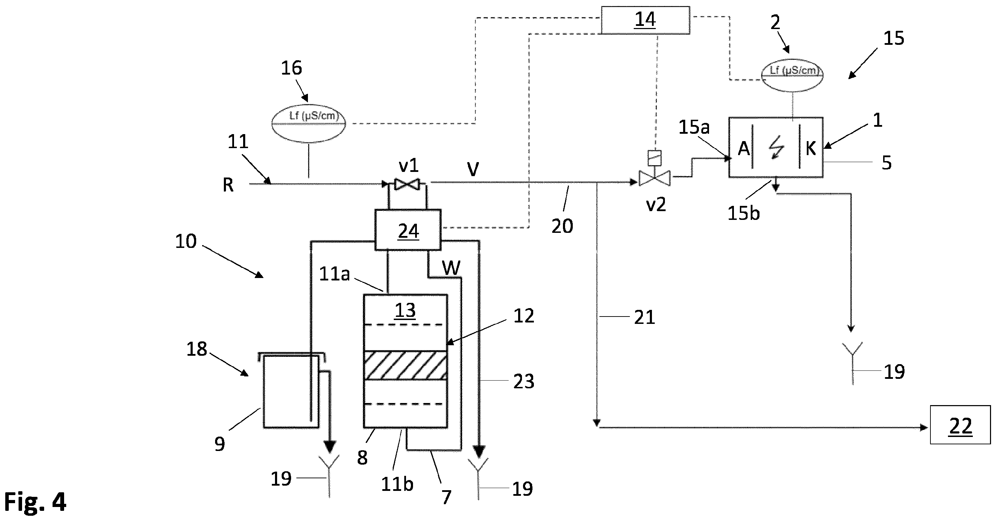

In Figur 4 ist ein Anwendungsbeispiel für das erfindungsgemäße Verfahren und die erfindungsgemäße Vorrichtung dargestellt, wobei eine erfindungsgemäße Vorrichtung mit einer Elektrolysezelle 1, die über einen Behälter 5 mit einem Eingang 15a und einem Ausgang 15b verfügt, in eine Wasserenthärtungseinrichtung 10 integriert ist. Die in Figur 4 schematisch gezeigte Wasserenthärtungseinrichtung 10 umfasst neben der erfindungsgemäßen Vorrichtung eine Enthärtungsvorrichtung 12, die einen regenerierbaren Ionenaustauscher 13 enthält. Der Ionenaustauscher 13 ist dabei in einem Ionenaustauscherbehälter 8, der einen Eingang 11a und einen Ausgang 11b aufweist, angeordnet. Zur Regenerierung des Ionenaustauschers 13 der Enthärtungsvorrichtung 12 umfasst die Wasserenthärtungseinrichtung 10 eine Regeneriereinrichtung 18, welche einen Regenerierbehälter 9 aufweist, in dem eine wässrige Regenerierlösung, insbesondere eine Natriumchlorid-Lösung, bevorratet ist. Bei einer Erschöpfung des Ionenaustauschers 13 der Enthärtungsvorrichtung 12 wird in einem Regenerierungsmodus die Regenerierlösung in und durch den Ionenaustauschbehälter 8 geleitet, wodurch der Ionenaustauscher 13 durch Austausch der darin beim Enthärten des Rohwassers R gebundenen Calcium- und Magnesium-Ionen durch Natrium-Ionen ersetzt werden. Die verbrauchte Regenerierlösung wird nach Beendigung des Regenerierungsmodus über eine Abfuhrleitung 23 in einen Kanal 19 geleitet.

Die Wasserenthärtungseinrichtung 10 umfasst ferner einen Rohwasserzulauf 11 zur Bereitstellung von Rohwasser R, wobei der Rohwasserzulauf 11 über einen Steuerkopf 24 mit dem Eingang 11a der Enthärtungsvorrichtung 12 verbunden ist. Zur Erfassung der Leitfähigkeit des zugeführten Rohwassers R ist in dem Rohwasserzulauf 11 ein Leitfähigkeitssensor 16 angeordnet. Zum Erzeugen von Verschnittwasser V ist ein regelbares Verschneideventil v1 vorgesehen, welches von der Steuereinrichtung 14 angesteuert werden kann. Der Steuerkopf 24 dient zur Steuerung der Volumenströme des Rohwassers R zur Enthärtungsvorrichtung 12 sowie des in der Enthärtungsvorrichtung 12 enthärteten Wassers W in eine mit dem Steuerkopf 24 in Verbindung stehende Verschnittwasserleitung 20 und wird hierfür ebenfalls von der Steuereinrichtung 14 angesteuert. Der Steuerkopf 24 bildet dabei zusammen mit dem Verschneideventil v1 eine Verschneideeinrichtung 24, v1, in der Verschnittwasser V durch Mischen von Rohwasser R aus dem Rohwasserzulauf 11 mit enthärtetem Rohwasser W aus der Enthärtungsvorrichtung 12 in einem durch die Stellung des regelbaren Ventils v1 vorgebbaren Mischungsverhältnis erzeugt wird.

Der Ausgang 11b der Enthärtungsvorrichtung 12 steht über eine Auslassleitung 7 mit dem Steuerkopf 24 in Verbindung, um das durch Ionenaustausch in dem Ionenaustauscher 13 enthärtete Wasser (Weichwasser W) in die Verschnittwasserleitung 20 zu leiten. In der Verschneideeinrichtung 24, v1 wird das enthärtete Wasser W in einem definierten Mischungsverhältnis mit Rohwasser R vermischt, um ein Verschnittwasser V zu erzeugen. Das Verschnittwasser V soll dabei eine vorgegebene Sollhärte aufweisen, beispielsweise von H = 5 °dH. Die Härte des Verschnittwassers V hängt dabei von der Härte des Rohwassers R und dem Mischungsverhältnis des enthärteten Wassers W mit dem Rohwasser R ab. Das in der Verschneideeinrichtung 24, v1 erzeugte Verschnittwasser V wird in der Verschnittwasserleitung 20 geführt und über eine mit der Verschnittwasserleitung 20 in Verbindung stehende Verbraucherleitung 21 zu einem Verbraucher 22 geleitet. Bei dem Verbraucher 22 kann es sich beispielsweise um die Trinkwasserinstallation eines Haushalts oder um ein Wassergerät handeln, dem direkt das Verschnittwasser zugeführt wird.

Zur Bestimmung und Einstellung eines geeigneten Mischungsverhältnisses, welches so gewählt ist, dass das Verschnittwasser V eine Härte aufweist, die möglichst gut der vorgegebenen Sollhärte, bspw. von 3°dH oder kleiner, entspricht, weist die Enthärtungseinrichtung eine Steuer- und Auswerteinrichtung 14 auf, welche mit der Verschneideeinrichtung 24, v1 gekoppelt ist, um durch Einstellung von regelbaren Ventilen der Verschneideeinrichtung 24, v1 das Mischungsverhältnis so einstellen zu können, dass die Härte des Verschnittwassers V der vorgegebenen Sollhärte entspricht. Zur Einstellung eines anfänglichen Mischungsverhältnisses, bei dem die Härte des Verschnittwassers V zumindest ungefähr der Sollhärte entspricht, ist die Steuer- und Auswerteinrichtung 14 mit dem im Rohwasserzulauf 11 angeordneten Leitfähigkeitssensor 16 gekoppelt und empfängt den von diesem Sensor erfassten Messwert der elektrischen Leitfähigkeit des Rohwassers R. Auf Basis des Messwerts der Leitfähigkeit des Rohwassers R berechnet die Steuer-und Auswerteinrichtung 14 durch Rückgriff auf eine Kennlinie, die den Verlauf der elektrischen Leitfähigkeit von Wasser in Abhängigkeit der Wasserhärte beschreibt, ein anfängliches Mischungsverhältnis.

Zur Feineinstellung des Mischungsverhältnisses wird die genaue Härte des Verschnittwassers V mittels der in die Enthärtungseinrichtung integrierten Vorrichtung zur Erfassung der Härte von Wasser bestimmt. Hierfür wird das Verschnittwasser V über die Verschnittwasserleitung 20 durch ein regelbares Ventil V2 zum Eingang 15a der Elektrolysezelle 1 geleitet. In der Elektrolysezelle 1 wird die elektrische Leitfähigkeit des Verschnittwassers V mittels des in die Elektrolysezelle 1 integrierten Leitfähigkeitssensors 2 gemessen und danach wird das in die Elektrolysezelle 1 eingeleitete Verschnittwasser V bei geschlossenem Behälter 5 der Elektrolysezelle 1 mittels der Elektrolyseelektroden A, K elektrolysiert, indem an die Elektrolyseelektroden A, K eine elektrische Gleichspannung angelegt wird. Dadurch werden die im Verschnittwasser V enthaltenen Erdalkali-Ionen aus dem Wasser ausgefällt. Während der elektrolytischen Fällung der Erdalkali-Ionen wird die elektrische Leitfähigkeit des sich im Behälter 5 der Elektrolysezelle 1 befindlichen Verschnittwassers V mit dem integrierten Leitfähigkeitssensor 2 gemessen, um den Umschlagspunkt U zu erfassen, der eine vollständige Ausfällung der Erdalkali-Ionen anzeigt, wie oben anhand der Vorrichtung von Figur 1 beschrieben. Nach vollständiger Ausfällung der Erdalkali-Ionen wird die elektrische Leitfähigkeit LF2 des elektrolysierten Verschnittwassers V gemessen und durch Differenzbildung mit der ursprünglichen Leitfähigkeit des Verschnittwassers V wird die durch das Fällen der Erdalkali-Ionen erzeugte Änderung der Leitfähigkeit ΔLf ermittelt. Hierfür ist die Steuer-und Auswerteinrichtung 14 mit dem Leitfähigkeitssensor 2 gekoppelt, um die Messwerte der Leitfähigkeitsmessung vor, während und nach der Fällung der Erdalkali-Ionen zu erhalten. Die Steuer- und Auswerteinrichtung 14 berechnet aus der erfassten Differenz der elektrischen Leitfähigkeit ΔLf unter Rückgriff auf den in einem Speicher der Steuer- und Auswerteinrichtung 14 hinterlegten Umrechnungsfaktor F die genaue Härte des Verschnittwassers V, wie oben beschrieben. Das in der Elektrolysezelle 1 elektrolysierte Verschnittwaser V kann nach Beendigung der Messung über einen Ausgang 15b aus dem Behälter 5 in einen Kanal 19 abgelassen werden.

Aus der bestimmten Härte des Verschnittwassers V kann über eine Härtebilanzrechnung bei bekanntem Mischungsverhältnis von Rohwasser R und enthärtetem Rohwasser bzw. dem Volumenanteilanteil von Rohwasser R im Verschnittwasser V auch die Gesamthärte des Rohwassers ermittelt werden. Dabei wird davon ausgegangen, dass das Verschnittwasser durch Mischung von Rohwasser und vollständig enthärtetem Rohwasser (Weichwasser), welches eine Gesamthärte von Null aufweist, erzeugt wird.

Auf Basis der genauen Härte des Verschnittwassers V, die von der Steuer- und Auswerteinrichtung 14 bestimmt worden ist, steuert die Steuer- und Auswerteinrichtung 14 die Verschneideeinrichtung 24, v1 an, um das Mischungsverhältnis des Rohwassers mit dem enthärteten Wasser W so zu justieren, dass das Verschnittwasser V eine Härte aufweist, die der vorgegebenen Sollhärte entspricht. Diese Nachjustierung des Mischungsverhältnisses erfolgt bevorzugt iterativ in einem Rückkopplungsprozess, um ein optimales Mischungsverhältnis einzustellen, bei dem die Härte des Verschnittwassers V möglichst genau der vorgegebenen Sollhärte entspricht. Dabei können auch Änderungen in der Härte des Rohwassers R, erkannt durch den Leitfähigkeitssensor 16, die bspw. auftreten können, wenn sich die Wasserqualität des von der öffentlichen Trinkwasserversorgung bereitgestellten Trinkwassers ändert, berücksichtigt werden, indem das Mischungsverhältnis an die geänderte Härte des Rohwassers R angepasst wird. Weiterhin kann aus der bestimmten Härte des Rohwassers R der Zeitpunkt einer Regenerierung der Wasserbehandlungsanlage gesteuert werden.

Wenn bspw. ein Rohwasser R mit einer Gesamthärte von $H_R$ = 15°dH über eine Enthärtungsanlage vollständig enthärtet und das enthärtete Rohwasser (Weichwasser mit 0°dH Gesamthärte) mit dem Rohwasser R zu einem Verschnittwasser V mit einem Rohwasseranteil von 1/3 vermischt wird, entsteht ein Verschnittwasser V mit einer Gesamthärte von $H_V$ = 15°dH · 1/3 = 5°dH. Wenn also die Gesamthärte $H_V$ eines Verschnittwassers V mit einem Rohwasseranteil von 1/n mit dem erfindungsgemäßen Verfahren bestimmt wird, kann daraus die Gesamthärte des Rohwassers $H_R$ = n · $H_V$ ermittelt werden.

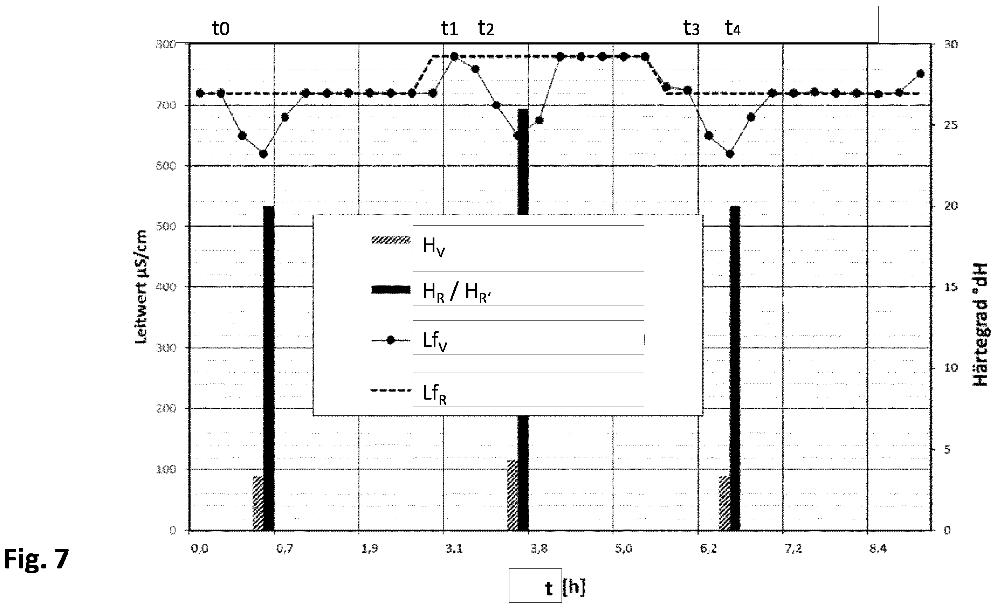

In Figur 7 ist beispielhaft der zeitliche Verlauf einer Messung der elektrischen Leitfähigkeit des Verschnittwassers V oder des Rohwassers R (gemessene Leitwerte $Lf_V$ bzw. $Lf_R$ in der Enthärtungseinrichtung der Figur 4 bei einer sich ändernden Härte des über den Rohwasserzulauf 10 zugeführten Rohwassers R sowie der zeitliche Verlauf der aus der Leitfähigkeit des Verschnittwassers V ermittelten Härte $H_V$ des Verschnittwassers V und der ermittelten Härte $H_R$ des Rohwassers R gezeigt. Dabei wurde der Enthärtungsvorrichtung 12 zwischen dem Beginn der Messung zum Zeitpunkt t = 0 ein Rohwasser R mit einer bestimmten Rohwasserhärte $H_R$ zugeführt. Ab dem Zeitpunkt $t_1$ wurde der Enthärtungs- vorrichtung 12 bis zum Zeitpunkt $t_3$ ein Rohwasser R' mit einer geänderten Zusammensetzung und einer höheren Härte $H_{R'}$ zugeführt und ab dem Zeitpunkt $t_3$ wurde wiederum das ursprüngliche Rohwasser R zugeführt. Dadurch kann eine in der Praxis häufig vorkommende plötzliche Änderung der Zusammensetzung bzw. der Wasserqualität und insbesondere der Härte von aus dem öffentlichem Trinkwassernetz zugeführtem Leitungswasser erfasst werden. Während des gesamten Messzeitraums zwischen t = 0 und t ≅ 9 h wurde kontinuierlich die Leitfähigkeit $Lf_R$ (t) des Rohwassers R bzw. R' mit dem Leitfähigkeitssensor 16 erfasst. Zum Zeitpunkt $t_1$ ist dabei aufgrund der höheren Härte des geänderten Rohwassers R' ein Anstieg der Leitfähigkeit $Lf_R$ (t) des Rohwassers zu beobachten. Ab dem Zeitpunkt $t_0$ wurde ein Messmodus durchgeführt um die Härte $H_V$ des Verschnittwassers V und daraus die Härte $H_R$ des Rohwassers R zu bestimmen und das Mischungsverhältnis von Rohwasser R mit enthärtetem Wasser W so einzustellen, dass die Härte $H_V$ des Verschnittwassers V einer vorgegebenen Sollhärte von ca. 3°dH entspricht. Aufgrund einer erfassten Änderung der Leitfähigkeit $Lf_R$ (t) des Rohwassers zum Zeitpunkt $t_1$ und $t_3$ wurde zu den Zeitpunkten $t_2$ und $t_4$ in der Elektrolysezelle 1 im Batchbetrieb eine Messung der Leitfähigkeit des Verschnittwassers und anschließend eine Fällung der Erdalkali-Ionen durch Elektrolyse des Verschnittwassers in der Elektrolysezelle eingeleitet und, wie oben beschrieben, die Leitfähigkeit $Lf_V$ des (elektrolysierten) Verschnittwassers und die Änderung der Leitfähigkeit $\Delta Lf$ während der Elektrolyse erfasst und daraus die Härte des Verschnittwassers $H_V$ sowie die Härte $H_{R'}$ des geänderten Rohwassers R' bestimmt.

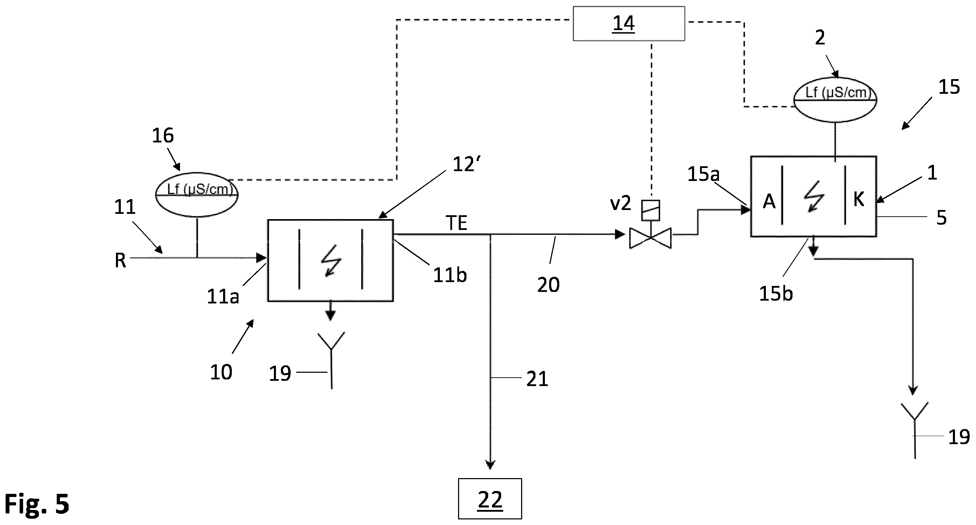

In Figur 5 ist ein weiteres Anwendungsbeispiel für die erfindungsgemäße Vorrichtung in einer Wasserbehandlungsanlage 10 schematisch dargestellt, wobei es sich bei der Wasserbehandlungsanlage 10 um ein membrankapazitives Deionisationsmodul (MCDI) 12' handelt, welches durch membrankapazitive Deionisation aus dem über den Rohwasserzulauf 11 zugeführten Rohwasser R teilentsalztes Wasser TE erzeugt, welches über eine Verbraucherleitung 21 zu einem Verbraucher 22 geleitet wird. Die Härte des teilentsalzten Wassers TE wird dabei wie in dem Anwendungsbeispiel der Figur 4 mittels einer erfindungsgemäßen Vorrichtung bestimmt, der das teilentsalzte Wasser TE über die Leitung 20 zugeführt wird. Im Übrigen entspricht der Aufbau und insbesondere die Ausgestaltung und die Funktion der Elektrolysezelle 1 dem Ausführungsbeispiel der Figur 4. Dabei kann das Deionisationsmodul (MCDI) 12' auch - wie die Enthärtungseinrichtung der Figur 4 - über eine Verschneideeinrichtung zur Herstellung von teilentsalztem Verschnittwasser V durch Vermischen von vollentsalztem Rohwasser mit Rohwasser verfügen, wobei die Regelung zur Einstellung der Härte des Verschnittwassers V auf eine vorgegebene Sollhärte dem Ausführungsbeispiel der Figur 4 entspricht.

In der nachfolgend aufgeführten **Tabelle 1** sind bevorzugte Bereiche und besonders bevorzugte Werte für die Parameter des erfindungsgemäßen Verfahrens angegeben:

**Tabelle 1**

| Parameter | Bevorzugte Bereiche | Besonders bevorzugte Parameter |
|---|---|---|
| **Elektrolysezelle** | | |
| Volumen | 5-200 ml | 18 ml |
| Elektrodenfläche der Elektrolyseelektro- den | 0,3 cm$^2$ (Stabelektroden) <br><br> bis 110 cm$^2$ (Flachelektroden) | 10 cm$^2$ |

(fortgesetzt)

| Parameter | Bevorzugte Bereiche | Besonders bevorzugte Parameter |
|---|---|---|
| **Elektrolysezelle** | | |
| Elektrodengeometrie der Elektrolysee-lektroden | Flachelektroden Stabelektroden Streckgitter | Flachelektroden |
| Anzahl der Elektrolyseelektroden | 2 | Je 1 Anode und 1 Kathode |
| Höhe x Breite x Dicke der Elektrolyseelektroden | | 15 x 67 x 1 mm |
| Material der Elektrolyseelektroden: Anode | oxidationsstabile Materialien | Titan platiniert (1,5μm) |
| Material der der Elektrolyseelektroden: Kathode | alkalibeständige Materialien | Graphitfolie 1 mm mit PTFE Anteil von 15% (PV15) Titan platiniert (1,5μm) |
| Abstand zwischen den Elektrolyseelektro-den | 1 mm - 30 mm | 18 mm |
| **Elektrische Parameter der** Elektrolyseelektroden | | |
| Stromart | Gleichstrom mit Konstantspannung (CV) oder Konstantstrom (CC) | CV |
| | | |
| Spannung | 2 - 15 V | CV: 5-14,5 V |
| Stromstärke | 5 - 400 mA | 20 - 100 mA |
| **Elektrische Parameter der Messelektroden des Leitfähigkeitssensors** | | |
| Stromart | Wechselstrom | |
| Spannung Wechselstrom | 5 - 20 V | 15 V |
| Frequenz Wechselstrom | 1 - 20 kHz | 2 kHz |

**Patentansprüche**

**1.** Verfahren zur Bestimmung der Härte von Wasser einer Wasserprobe, welche Erdalkali-Ionen, insbesondere Calcium- und/oder Magnesium-Ionen, sowie im Überschuss zu den Erdalkali-Ionen Hydrogencarbonat-Ionen enthält, mit folgenden Schritten:

- Erfassung der ursprünglichen elektrischen Leitfähigkeit ($Lf_1$) der Wasserprobe,
- Fällung der Erdalkali-Ionen durch Elektrolyse der Wasserprobe in einer Elektrolysezelle,
- Erfassung der Leitfähigkeit ($Lf_2$) der Wasserprobe nach einer vollständigen oder zumindest weitgehend vollständigen Fällung der Erdalkali-Ionen und Erfassung der durch die Fällung der Erdalkali-Ionen hervor-gerufenen Änderung der Leitfähigkeit ($\Delta Lf = |Lf_2 - Lf_1|$) der Wasserprobe,
- Bestimmung der Gesamthärte des Wassers der Wasserprobe aus der erfassten Änderung der Leitfähigkeit ($\Delta Lf$) und einem vorgegebenen Umrechnungsfaktor (F) oder einer vorgegebenen Kennlinie des Verlaufs der Leitfähigkeitsänderung von Wasser in Abhängigkeit der Härteänderung des Wassers bei einer Ausfällung der Carbonathärte.

**2.** Verfahren nach Anspruch 1, **dadurch gekennzeichnet, dass** die Wasserprobe zur Einstellung des Überschusses der Hydrogencarbonat-Ionen gegenüber den Erdalkali-Ionen vor der Fällung der Erdalkali-Ionen durch Vermischung mit enthärtetem Wasser und/oder durch Zugabe von Hydrogencarbonat-Ionen und/oder Carbonat-Ionen aufbereitet wird.

3. Verfahren nach einem der voranstehenden Ansprüche, **dadurch gekennzeichnet, dass** der Umrechnungsfaktor (F) ein Proportionalitätsfaktor für einen linearen Zusammenhang zwischen der Leitfähigkeit von Wasser und der absoluten Gesamthärte oder der absoluten Carbonathärte des Wassers ist und insbesondere den linearen Verlauf der Änderung der elektrischen Leitfähigkeit einer Wasserprobe in Abhängigkeit der Härteänderung der Wasserprobe auf einer Härteskala, insbesondere der deutschen Wasserhärte, bei einer Ausfällung der Carbonathärte charakterisiert.

4. Verfahren nach einem der voranstehenden Ansprüche, **dadurch gekennzeichnet, dass** die Wasserprobe durch Vermischen von Rohwasser (R) mit enthärtetem Rohwasser (W) in einem vorgegebenen Mischungsverhältnis erzeugt wird, wobei die Gesamthärte des Rohwassers (R) unter Berücksichtigung des Mischungsverhältnisses aus der erfassten Änderung der Leitfähigkeit ($\Delta$Lf) der Wasserprobe ermittelt wird.

5. Verfahren nach einem der voranstehenden Ansprüche, **dadurch gekennzeichnet, dass** die Leitfähigkeit der Wasserprobe während der elektrolytischen Fällung der Erdalkali-Ionen bis zu einem Umschlagpunkt (U) erfasst wird, bei dem die erfasste Leitfähigkeit aufgrund einer zumindest weitgehend vollständigen Fällung der Erdalkali-Ionen einen Minimalwert ($Lf_2$) aufweist und optional danach bei weiterer Elektrolyse der Wasserprobe ein Anstieg der erfassten Leitfähigkeit der Wasserprobe beobachtet wird, wobei die Leitfähigkeit der Wasserprobe während der elektrolytischen Fällung der Erdalkali-Ionen insbesondere solange erfasst wird, bis mindestens 90% der Erdalkali-Ionen, bevorzugt mehr als 95%, durch die Elektrolyse ausgefällt sind.

6. Verfahren nach einem der voranstehenden Ansprüche, **dadurch gekennzeichnet, dass** die Wasserprobe zur Fällung der Erdalkali-Ionen in eine Elektrolysezelle (1) eingebracht wird, wobei die Elektrolysezelle (1) mindestens zwei Elektrolyseelektroden (A, K), welche zur elektrolytischen Fällung der Erdalkali-Ionen mit Gleichstrom beaufschlagt werden, und wenigstens einen Leitfähigkeitssensor (2) enthält, wobei der wenigstens eine Leitfähigkeitssensor (2) in die Elektrolysezelle (1) integriert ist oder ein erster Leitfähigkeitssensor (2a) an einem Eingang (15a) und ein zweiter Leitfähigkeitssensor (2b) an einem Ausgang (15b) der Elektrolysezelle (1) angeordnet ist.

7. Verfahren nach Anspruch 6, **dadurch gekennzeichnet, dass** die Wasserprobe zur Fällung der Erdalkali-Ionen im Batchbetrieb in die Elektrolysezelle (1) eingebracht wird, wobei in der Elektrolysezelle (1) zeitlich abwechselnd eine elektrolytische Fällung der Erdalkali-Ionen und eine Messung der Leitfähigkeit der Wasserprobe erfolgt.

8. Verfahren nach einem der Ansprüche 6 oder 7, wobei in einem Messmodus eine Gleichspannung an die Elektrolyseelektroden (A, K) mit einer ersten Polarität angelegt wird und in einem Regeneriermodus, der insbesondere nach einer bestimmten Betriebsdauer der Vorrichtung und/oder einer bestimmten Anzahl an durchgeführten Messzyklen oder zu bestimmten Zeiten eingeleitet wird, eine Gleichspannung an die Elektrolyseelektroden (A, K) mit einer zur ersten Polarität entgegengesetzten Polarität angelegt wird.

9. Verfahren nach einem der voranstehenden Ansprüche, **dadurch gekennzeichnet, dass** während der elektrolytischen Fällung der Erdalkali-Ionen die Temperatur der Wasserprobe gemessen und bei der Bestimmung der Gesamthärte des Wassers der Wasserprobe die erfasste Änderung der Leitfähigkeit ($\Delta$Lf) einer Temperaturkorrektur über einen Temperaturkorrekturfaktor oder eine Temperaturkennlinie unterzogen wird.

10. Vorrichtung zur Erfassung der Härte von Wasser einer Wasserprobe, welche Erdalkali-Ionen, insbesondere Calcium- und/oder Magnesium-Ionen, sowie im Überschuss zu den Erdalkali-Ionen Hydrogencarbonat-Ionen enthält, wobei die Vorrichtung eine Elektrolysezelle (1) mit wenigstens zwei Elektrolyseelektroden (A, K), mindestens einen Leitfähigkeitssensor (2), sowie eine Steuer- und Auswerteeinrichtung umfasst, wobei die Steuer- und Auswerteeinrichtung so eingerichtet ist, dass sie zunächst die ursprüngliche elektrische Leitfähigkeit ($Lf_1$) der Wasserprobe erfasst, danach die Erdalkali-Ionen der Wasserprobe durch Anlegen einer Gleichspannung an die Elektrolyseelektroden (A, K) mit einer ersten Polarität zumindest teilweise und bevorzugt vollständig oder zumindest weitgehend vollständig ausgefällt werden und schließlich die Leitfähigkeit ($Lf_2$) der Wasserprobe während und/oder nach der Fällung der Erdalkali-Ionen erfasst und den Betrag der Differenz der ursprünglichen Leitfähigkeit und der nach der Fällung der Erdalkali-Ionen gemessenen Leitfähigkeit ($\Delta$Lf = $|Lf_2 - Lf_1|$) ermittelt.

11. Vorrichtung nach Anspruch 10, **dadurch gekennzeichnet, dass** die Elektrolyseelektroden (A, K) als in der Elektrolysezelle (1) gegenüberliegend angeordnete Flachelektroden ausgebildet sind, wobei die Elektrolyseelektroden (A, K) an eine Gleichstromquelle angeschlossen oder anschließbar sind und die Messelektroden bevorzugt zwischen den einander gegenüberliegenden Elektrolyseelektroden (A, K) der Elektrolysezelle (1) angeordnet und insbesondere als Stabelektroden ausgebildet sind.

**12.** Vorrichtung nach Anspruch 10 oder 11, **dadurch gekennzeichnet, dass** der wenigstens eine Leitfähigkeitssensor (2) in die Elektrolysezelle (1) integriert ist oder ein erster Leitfähigkeitssensor (2a) an einem Eingang (15a) und ein zweiter Leitfähigkeitssensor (2b) an einem Ausgang (15b) der Elektrolysezelle (1) angeordnet ist, wobei der in der Elektrolysezelle (1) integrierte Leitfähigkeitssensor (2) oder jeder Leitfähigkeitssensor (2a, 2b) bevorzugt mindestens ein Elektrodenpaar (3) mit zwei Messelektroden umfasst, wobei die Messelektroden an eine Wechselspannungsquelle angeschlossen oder anschließbar sind.

**13.** Vorrichtung nach einem der Ansprüche 10 bis 12, **dadurch gekennzeichnet, dass** zwischen zwei gegenüberliegenden Elektrolyseelektroden (A, K) eine Kationenaustauschermembran (KAT) angeordnet ist, wobei die Kationenaustauschermembran (KAT) insbesondere parallel zu den als Flachelektroden ausgebildeten Elektrolyseelektroden (A, K) verläuft.

**14.** Vorrichtung nach einem der Ansprüche 10 bis 13, **dadurch gekennzeichnet, dass** die Steuer- und Auswerteeinrichtung einen Datenspeicher enthält, in dem ein Umrechnungsfaktor (F) und/oder eine Kennlinie des Verlaufs der Leitfähigkeit von Wasser in Abhängigkeit der Gesamthärte oder der Carbonathärte des Wassers gespeichert ist, wobei die Steuer- und Auswerteeinrichtung so eingerichtet ist, dass sie in einem Messmodus auf den abgespeicherten Umrechnungsfaktor (F) oder die Kennlinie zugreift und aus der erfassten Änderung der Leitfähigkeit ($\Delta$Lf) und dem Umrechnungsfaktor (F) oder der Kennlinie die Gesamthärte des Wassers auf einer dem Umrechnungsfaktor (F) oder der Kennlinie zugeordneten Härteskala berechnet, und die Steuer- und Auswerteeinrichtung bevorzugt so eingerichtet ist, dass sie in einem Regeneriermodus, der insbesondere nach einer bestimmten Betriebsdauer der Vorrichtung und/oder einer bestimmten Anzahl an durchgeführten Messzyklen oder zu bestimmten Zeiten eingeleitet wird, eine Gleichspannung an die Elektrolyseelektroden (A, K) mit einer zur ersten Polarität entgegengesetzten Polarität anlegt.

**15.** Verwendung der Vorrichtung nach einem der Ansprüche 10 bis 14 in einer Wasserbehandlungsanlage, insbesondere in einer Wasserenthärtungsanlage oder einer Entsalzungsanlage und insbesondere in einer Umkehrosmoseanlage (RO) oder einer membrankapazitiven Deionisationsanlage (MCDI) oder einer Nanofiltrationsanlage (NF), zur Bestimmung der Härte von Rohwasser, das der Wasserbehandlungsanlage zugeführt wird, und/oder zur Bestimmung der Härte von in der Wasserbehandlungsanlage behandeltem, insbesondere enthärtetem oder entsalztem Wasser.

**Claims**

**1.** Method for determining the hardness of water in a water sample containing alkaline earth ions, in particular calcium and/or magnesium ions, as well as hydrogen carbonate ions in excess of the alkaline earth ions, comprising the following steps:

- measuring the original electrical conductivity ($Lf_1$) of the water sample,
- precipitating the alkaline earth ions by electrolysis of the water sample in an electrolysis cell,
- detecting the conductivity ($Lf_2$) of the water sample after a complete or at least largely complete precipitation of the alkaline earth ions and detecting the change in conductivity ($\Delta Lf = |Lf_2 - Lf_1|$) of the water sample caused by the precipitation of the alkaline earth ions,
- determination of the total hardness of the water in the water sample from the measured change in conductivity ($\Delta$Lf) and a predetermined conversion factor (F) or a predetermined characteristic curve of the change in conductivity of water as a function of the change in hardness of the water upon precipitation of the carbonate hardness.

**2.** Method according to claim 1, **characterised in that** the water sample is treated to adjust the excess of hydrogen carbonate ions relative to the alkaline earth ions before the precipitation of the alkaline earth ions by mixing with softened water and/or by adding hydrogen carbonate ions and/or carbonate ions.

**3.** Method according to any one of the preceding claims, **characterised in that** the conversion factor (F) is a proportionality factor for a linear relationship between the conductivity of water and the absolute total hardness or the absolute carbonate hardness of the water and, in particular, characterises the linear course of the change in the electrical conductivity of a water sample as a function of the change in hardness of the water sample on a hardness scale, in particular the German water hardness, in the event of precipitation of the carbonate hardness.

**4.** Method according to any one of the preceding claims, **characterised in that** the water sample is produced by mixing raw water (R) with softened raw water (W) in a predetermined mixing ratio, whereby the total hardness of the raw water (R) is determined from the recorded change in conductivity ($\Delta Lf$) of the water sample, taking into account the mixing ratio.

5. Method according to one of the preceding claims, **characterised in that** the conductivity of the water sample is measured during the electrolytic precipitation of the alkaline earth ions up to an inflection point (U) at which the measured conductivity exhibits a minimum value ($Lf_2$) due to an at least largely complete precipitation of the alkaline earth ions, and optionally thereafter, during further electrolysis of the water sample, an increase in the detected conductivity of the water sample is observed, wherein the conductivity of the water sample is detected during the electrolytic precipitation of the alkaline earth ions, in particular until at least 90% of the alkaline earth ions, preferably more than 95%, have been precipitated by the electrolysis.

6. Method according to any one of the preceding claims, **characterised in that** the water sample is introduced into an electrolysis cell (1) for the precipitation of the alkaline earth ions, wherein the electrolysis cell (1) contains at least two electrolysis electrodes (A, K), which are supplied with direct current for the electrolytic precipitation of the alkaline earth ions, and at least one conductivity sensor (2), wherein the at least one conductivity sensor (2) is integrated into the electrolysis cell (1) or a first conductivity sensor (2a) is arranged at an inlet (15a) and a second conductivity sensor (2b) is arranged at an outlet (15b) of the electrolysis cell (1).

7. Method according to claim 6, **characterised in that** the water sample is introduced into the electrolysis cell (1) in a batch mode for the precipitation of the alkaline earth ions, wherein an electrolytic precipitation of the alkaline earth ions and a measurement of the conductivity of the water sample are performed alternately in the electrolysis cell (1).

8. Method according to one of claims 6 or 7, wherein in a measurement mode, a direct voltage is applied to the electrolysis electrodes (A, K) with a first polarity and, in a regeneration mode, which is initiated in particular after a certain operating time of the device and/or a certain number of measurement cycles performed or at certain times, a direct voltage is applied to the electrolysis electrodes (A, K) with a polarity opposite to the first polarity.

9. Method according to any one of the preceding claims, **characterised in that** during the electrolytic precipitation of the alkaline earth ions, the temperature of the water sample is measured and, when determining the total hardness of the water sample, the detected change in conductivity ($\Delta Lf$) is subjected to a temperature correction via a temperature correction factor or a temperature characteristic curve.

10. Device for measuring the hardness of water in a water sample containing alkaline earth ions, in particular calcium and/or magnesium ions, as well as hydrogen carbonate ions in excess of the alkaline earth ions, wherein the device comprises an electrolysis cell (1) with at least two electrolysis electrodes (A, K), at least one conductivity sensor (2), and a control and evaluation device, wherein the control and evaluation device is designed such that it first detects the original electrical conductivity ($Lf_1$) of the water sample, then at least partially and preferably completely or at least largely completely precipitates the alkaline earth ions of the water sample by applying a direct voltage with a first polarity to the electrolysis electrodes (A, K), and finally detects the conductivity ($Lf_2$) of the water sample during and/or after the precipitation of the alkaline earth ions and determines the amount of the difference between the original conductivity and the conductivity measured after the precipitation of the alkaline earth ions ($\Delta Lf = |Lf_2 - Lf_1|$).

11. Device according to claim 10, **characterised in that** the electrolysis electrodes (A, K) are designed as flat electrodes arranged opposite each other in the electrolysis cell (1), wherein the electrolysis electrodes (A, K) are connected or can be connected to a direct current source and the measuring electrodes are preferably arranged between the opposing electrolysis electrodes (A, K) of the electrolysis cell (1) and are preferably designed as rod electrodes.

12. Device according to claim 10 or 11, **characterised in that** the at least one conductivity sensor (2) is integrated into the electrolysis cell (1) or a first conductivity sensor (2a) is arranged at an inlet (15a) and a second conductivity sensor (2b) is arranged at an outlet (15b) of the electrolysis cell (1), wherein the conductivity sensor (2) integrated in the electrolysis cell (1) or each conductivity sensor (2a, 2b) preferably comprises at least one electrode pair (3) with two measuring electrodes, wherein the measuring electrodes are connected or connectable to an AC voltage source.

13. Device according to any one of claims 10 to 12, **characterised in that** a cation exchange membrane (KAT) is arranged between two opposing electrolysis electrodes (A, K), wherein the cation exchange membrane (KAT) runs in particular parallel to the electrolysis electrodes (A, K) designed as flat electrodes.

**14.** Device according to any one of claims 10 to 13, **characterised in that** the control and evaluation device contains a data memory in which a conversion factor (F) and/or a characteristic curve of the conductivity of water as a function of the total hardness or carbonate hardness of the water is stored, wherein the control and evaluation device is set up such that, in a measurement mode, accesses the stored conversion factor (F) stored therein and calculates the total hardness of the water on a hardness scale assigned to the conversion factor (F) or the characteristic curve from the detected change in conductivity ($\Delta$Lf) and the conversion factor (F) or the characteristic curve, and the control and evaluation device is preferably configured such that, in a regeneration mode which is initiated in particular after a certain operating period of the device and/or a certain number of measurement cycles performed or at certain times, applies a direct voltage to the electrolysis electrodes (A, K) with a polarity opposite to the first polarity.

**15.** Use of the device according to any one of claims 10 to 14 in a water treatment plant, in particular in a water softening plant or a desalination plant and in particular in a reverse osmosis plant (RO) or a membrane capacitive deionisation plant (MCDI) or a nanofiltration plant (NF), for determining the hardness of raw water supplied to the water treatment plant and/or for determining the hardness of water treated in the water treatment plant, in particular softened or desalinated water.


**Revendications**

**1.** Procédé permettant de déterminer la dureté de l'eau d'un échantillon d'eau qui contient des ions alcalino-terreux, en particulier des ions calcium et/ou des ions magnésium, ainsi que des ions hydrogénocarbonate en excès par rapport aux ions alcalino-terreux, comportant les étapes suivantes :

- détection de la conductivité électrique initiale ($Lf_1$) de l'échantillon d'eau,
- précipitation des ions alcalino-terreux par électrolyse de l'échantillon d'eau dans une cellule d'électrolyse,
- détection de la conductivité ($Lf_2$) de l'échantillon d'eau après une précipitation complète ou au moins largement complète des ions alcalino-terreux et détection de la modification de la conductivité ($\Delta Lf = |Lf_2 - Lf_1|$) de l'échantillon d'eau provoquée par la précipitation des ions alcalino-terreux,
- détermination de la dureté totale de l'eau de l'échantillon d'eau à partir de la modification détectée de la conductivité ($\Delta$Lf) et d'un facteur de conversion (F) prédéfini ou d'une courbe caractéristique prédéfinie de l'évolution de la modification de la conductivité de l'eau en fonction de la modification de la dureté de l'eau lors d'une précipitation de la dureté carbonatée.

**2.** Procédé selon la revendication 1, **caractérisé en ce que,** pour ajuster l'excès d'ions hydrogénocarbonate par rapport aux ions alcalino-terreux, l'échantillon d'eau est traité, avant la précipitation des ions alcalino-terreux, par mélange avec de l'eau adoucie et/ou par addition d'ions hydrogénocarbonate et/ou d'ions carbonate.

**3.** Procédé selon l'une des revendications précédentes,
**caractérisé en ce que** le facteur de conversion (F) est un facteur de proportionnalité pour une relation linéaire entre la conductivité de l'eau et la dureté totale absolue ou la dureté carbonatée absolue de l'eau et caractérise en particulier l'évolution linéaire de la modification de la conductivité électrique d'un échantillon d'eau en fonction de la modification de la dureté de l'échantillon d'eau sur une échelle de dureté, en particulier de la dureté de l'eau allemande, lors d'une précipitation de la dureté carbonatée.

**4.** Procédé selon l'une des revendications précédentes,
**caractérisé en ce que** l'échantillon d'eau est produit en mélangeant de l'eau brute (R) avec de l'eau brute adoucie (W) dans un rapport de mélange prédéfini, dans lequel la dureté totale de l'eau brute (R) est déterminée à partir de la modification détectée de la conductivité ($\Delta$Lf) de l'échantillon d'eau en tenant compte du rapport de mélange.

**5.** Procédé selon l'une des revendications précédentes,
**caractérisé en ce que** la conductivité de l'échantillon d'eau est détectée pendant la précipitation électrolytique des ions alcalino-terreux jusqu'à un point d'inversion (U) auquel la conductivité détectée présente une valeur minimale ($Lf_2$) en raison d'une précipitation au moins largement complète des ions alcalino-terreux et, éventuellement, une augmentation de la conductivité détectée de l'échantillon d'eau est ensuite observée lors d'une électrolyse supplémentaire de l'échantillon d'eau, dans lequel la conductivité de l'échantillon d'eau est détectée pendant la précipitation électrolytique des ions alcalino-terreux, en particulier jusqu'à ce qu'au moins 90 % des ions alcalino-terreux, de préférence plus de 95 %, soient précipités par l'électrolyse.

6. Procédé selon l'une des revendications précédentes, **caractérisé en ce que** l'échantillon d'eau est introduit dans une cellule d'électrolyse (1) pour la précipitation des ions alcalino-terreux, dans lequel la cellule d'électrolyse (1) comprend au moins deux électrodes d'électrolyse (A, K) qui sont alimentées en courant continu pour la précipitation électrolytique des ions alcalino-terreux, et au moins un capteur de conductivité (2), dans lequel l'au moins un capteur de conductivité (2) est intégré dans la cellule d'électrolyse (1) ou un premier capteur de conductivité (2a) est disposé à une entrée (15a) de la cellule d'électrolyse (1) et un second capteur de conductivité (2b) est disposé à une sortie (15b) de la cellule d'électrolyse.

7. Procédé selon la revendication 6, **caractérisé en ce que** l'échantillon d'eau est introduit dans la cellule d'électrolyse (1) pour la précipitation des ions alcalino-terreux dans le mode de fonctionnement discontinu, dans lequel une précipitation électrolytique des ions alcalino-terreux et une mesure de la conductivité de l'échantillon d'eau sont effectuées en alternance dans le temps dans la cellule d'électrolyse (1).

8. Procédé selon l'une des revendications 6 ou 7, dans lequel, dans un mode de mesure, une tension continue est appliquée aux électrodes d'électrolyse (A, K) avec une première polarité et, dans un mode de régénération, initié en particulier après une durée de fonctionnement déterminée du dispositif et/ou un nombre déterminé de cycles de mesure réalisés ou à des instants déterminés, une tension continue est appliquée aux électrodes d'électrolyse (A, K) avec une polarité opposée à la première polarité.

9. Procédé selon l'une des revendications précédentes, **caractérisé en ce que,** pendant la précipitation électrolytique des ions alcalino-terreux, la température de l'échantillon d'eau est mesurée et, lors de la détermination de la dureté totale de l'eau de l'échantillon d'eau, la modification détectée de la conductivité ($\Delta Lf$) est soumise à une correction de température par l'intermédiaire d'un facteur de correction de température ou d'une courbe caractéristique de température.

10. Dispositif permettant de détecter la dureté de l'eau d'un échantillon d'eau qui contient des ions alcalino-terreux, en particulier des ions calcium et/ou des ions magnésium, ainsi que des ions hydrogénocarbonate en excès par rapport aux ions alcalino-terreux, dans lequel le dispositif comprend une cellule d'électrolyse (1) comportant au moins deux électrodes d'électrolyse (A, K), au moins un capteur de conductivité (2), ainsi qu'un appareil de commande et d'évaluation, dans lequel l'appareil de commande et d'évaluation est configuré de sorte qu'il détecte d'abord la conductivité électrique initiale ($Lf_1$) de l'échantillon d'eau, ensuite, les ions alcalino-terreux de l'échantillon d'eau sont précipités au moins partiellement et de préférence complètement ou au moins en grande partie complètement par l'application d'une tension continue aux électrodes d'électrolyse (A, K) avec une première polarité, et enfin, il détecte la conductivité ($Lf_2$) de l'échantillon d'eau pendant et/ou après la précipitation des ions alcalino-terreux et détermine la valeur de la différence entre la conductivité initiale et la conductivité mesurée après la précipitation des ions alcalino-terreux ($\Delta Lf = |Lf_2 - Lf_1|$).

11. Dispositif selon la revendication 10, **caractérisé en ce que** les électrodes d'électrolyse (A, K) sont conçues sous forme d'électrodes plates disposées en se faisant face dans la cellule d'électrolyse (1), dans lequel les électrodes d'électrolyse (A, K) sont raccordées ou peuvent être raccordées à une source de courant continu et les électrodes de mesure sont disposées de préférence entre les électrodes d'électrolyse (A, K) de la cellule d'électrolyse (1) se faisant face et sont conçues en particulier sous forme d'électrodes en baguette.

12. Dispositif selon la revendication 10 ou 11, **caractérisé en ce que** l'au moins un capteur de conductivité (2) est intégré dans la cellule d'électrolyse (1) ou un premier capteur de conductivité (2a) est disposé à une entrée (15a) de la cellule d'électrolyse (1) et un second capteur de conductivité (2b) est disposé à une sortie (15b) de la cellule d'électrolyse, dans lequel le capteur de conductivité (2) intégré dans la cellule d'électrolyse (1) ou chaque capteur de conductivité (2a, 2b) comprend de préférence au moins une paire d'électrodes (3) comportant deux électrodes de mesure, dans lequel les électrodes de mesure sont raccordées ou peuvent être raccordées à une source de tension alternative.

13. Dispositif selon l'une des revendications 10 à 12, **caractérisé en ce qu'**une membrane échangeuse de cations (KAT) est disposée entre deux électrodes d'électrolyse (A, K) se faisant face, dans lequel la membrane échangeuse de cations (KAT) s'étend en particulier parallèlement aux électrodes d'électrolyse (A, K) conçues sous forme d'électrodes plates.

14. Dispositif selon l'une des revendications 10 à 13, **caractérisé en ce que** l'appareil de commande et d'évaluation comprend une mémoire de données dans laquelle sont mémorisés un facteur de conversion (F) et/ou une courbe caractéristique de l'évolution de la conductivité de l'eau en fonction de la dureté totale ou de la dureté carbonatée de l'eau, dans lequel l'appareil de commande et d'évaluation est configuré de sorte qu'il accède, dans un mode de mesure, au facteur de conversion (F) mémorisé ou à la courbe caractéristique mémorisée et calcule la dureté totale de l'eau sur une échelle de dureté associée au facteur de conversion (F) ou à la courbe caractéristique à partir de la modification détectée de la conductivité (ΔLf) et du facteur de conversion (F) ou de la courbe caractéristique, et l'appareil de commande et d'évaluation est de préférence configuré de sorte que, dans un mode de régénération qui est en particulier déclenché après une durée de fonctionnement déterminée du dispositif et/ou un nombre déterminé de cycles de mesure réalisés ou à des instants déterminés, il applique une tension continue aux électrodes d'électrolyse (A, K) avec une polarité opposée à la première polarité.

15. Utilisation du dispositif selon l'une des revendications 10 à 14 dans une installation de traitement d'eau, en particulier dans une installation d'adoucissement d'eau ou une installation de déminéralisation et en particulier dans une installation d'osmose inverse (RO) ou une installation de déionisation capacitive membranaire (MCDI) ou une installation de nanofiltration (NF), pour la détermination de la dureté de l'eau brute qui est amenée à l'installation de traitement d'eau et/ou pour la détermination de la dureté de l'eau traitée, en particulier adoucie ou déminéralisée, dans l'installation de traitement d'eau.

**Fig. 1**

**Fig. 2**

**Fig. 3**

EP 4 500 173 B1

Fig. 4

Fig. 5

Fig. 6

Fig. 7

**IN DER BESCHREIBUNG AUFGEFÜHRTE DOKUMENTE**

**In der Beschreibung aufgeführte Patentdokumente**

- EP 2169392 B1
- DE 102016100195 A1